# EUROPÄISCHE PATENTSCHRIFT

(11) **EP 1 747 208 B1**
(45) Veröffentlichungstag und Bekanntmachung des Hinweises auf die Patenterteilung: **25.09.2013**
(21) Anmeldenummer: 05740022.8
(22) Anmeldetag: 10.05.2005
(51) Int. Cl.: C07D 265/36, C07D 471/08, A61K 31/538, A61P 11/00

(54) **NEUE LANGWIRKSAME BRONCHODILATOREN ZUR BEHANDLUNG VON ATEMWEGSERKRANKUNGEN**
NOVEL SUSTAINED-ACTION BRONCHODILATING AGENTS FOR USE IN THE TREATMENT OF RESPIRATORY DISEASES
NOUVEAUX BRONCHODILATATEURS POUR LE TRAITEMENT DE MALADIES RESPIRATOIRES

(30) Priorität: 14.05.2004 DE 102004024453
(43) Veröffentlichungstag der Anmeldung: 31.01.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: BOUYSSOU, Thierry, 88447 Warthhausen (DE); SCHNAPP, Andreas, 88400 Biberach (DE); LUSTENBERGER, Philipp, CH-4056 Basel (DE); RUDOLF, Klaus, 88447 Warthausen (DE); PIEPER, Michael, P., 88400 Biberach (DE); GRAUERT, Matthias, 88400 Biberach (DE); BREITFELDER, Steffen, 88433 Assmannshardt (DE); SPECK, Georg, 55218 Ingelheim (DE)
(74) Vertreter: Simon, Elke Anna Maria
(86) Internationale Anmeldenummer: PCT/EP2005/005026
(87) Internationale Veröffentlichungsnummer: WO 2005/111004

(56) Entgegenhaltungen:
- EP-A- 0 073 505
- EP-A- 0 321 864
- EP-A- 1 577 306
- WO-A-01/83462
- WO-A-02/32897
- WO-A-2004/022058
- WO-A-2004/045618
- DE-A1- 10 256 317
- US-A- 4 460 581
- MILECKI J ET AL: "CARBOSTYRIL DERIVATIVES HAVING POTENT BETA-ADRENERGIC AGONIST PROPERTIES" 1. September 1987 (1987-09-01), JOURNAL OF MEDICINAL CHEMISTRY, AMERICAN CHEMICAL SOCIETY. WASHINGTON, US, PAGE(S) 1563-1566 , XP000644541 ISSN: 0022-2623 Formel 13

## Beschreibung

Gegenstand der Erfindung sind Verbindungen der allgemeinen Formel **1** 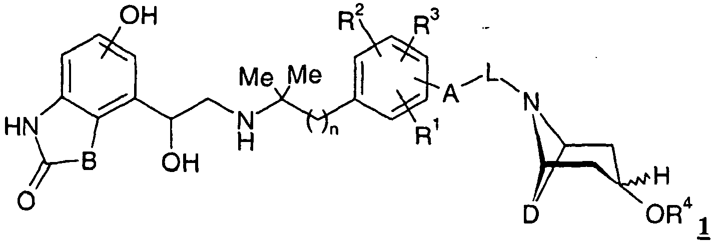 worin n, A, B, D, L, R¹, R², R³ und R⁴ die in den Ansprüchen und in der Beschreibung genannten Bedeutungen haben können, Verfahren zu deren Herstellung, sowie deren Verwendung als Arzneimittel, insbesondere als Arzneimittel zur Behandlung von Atemwegserkrankungen.

Die Patentschriften bzw. Patentanmeldungen US4, 460, 581, EP073505 A1, EP 321864 A2, und WO01/83462 A1 beschreiben Benzoxazinderivate zur Behandlung von Asthma und Bronchitis.

### Beschreibung der Erfindung

Gegenstand der Erfindung sind Verbindungen der allgemeinen Formel **1** 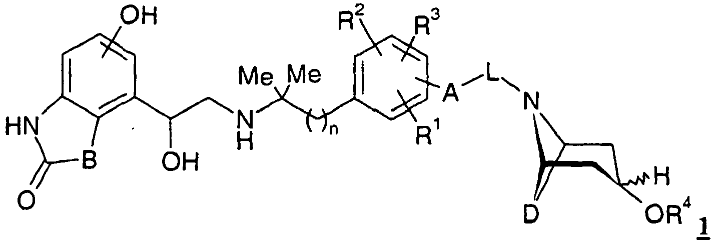 worin
- n: 1, 2 oder 3, bevorzugt 1 und 2;
- A: O, N oder eine Einfachbindung;
- B: ein zweibindiger Rest ausgewählt aus der Gruppe bestehend aus -CH₂-CH₂-, -CH=CH- und -CH₂-O-;
- L: ein zweibindiger Rest ausgewählt aus der Gruppe bestehend aus -CO-(CH₂)ₘ-, -CO-(CH₂)ₘ-NH-CO-(CH₂)ₒ-, -CO-(CH₂)ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ-, -(CH₂)ₚ-CO-NH-(CH₂)ₒ-, S0₂-(CH₂)ₘ-, -SO₂-(CH₂),-NH-CO-(CH₂)ₒ-, -SO₂-(CH₂)ₘ-CO-NH-(CH₂)ₒ-, -SO₂-(CH₂)ₘ-O-(CH₂)ₚ-;
- m, o: unabhängig voneinander 1 bis 12;
- p, r: unabhängig voneinander 2 bis 12;
- R¹, R^{2,} R³: gleich oder verschieden, Wasserstoff, C₁-C₆-Alkyl, Halogen-C₁-C₆-alkylen, OH, -CF₃, CHF₂, HO-C₁₋₆-alkylen, -O-C₁-C₆-Alkyl, C₆-C₁₀-Aryl, C₆-C₁₀-Aryl-C₁-C₄-alkylen, C₆-C₁₀-Aryl-C₁-C₆-alkylen-O-, -COOH, -COOC₁-C₆-alkyl, -O-C₁-C₆-alkylen-COOH, -O-C₁-C₆-alkylen-COOC₁-C₆-alkyl, -NHSO₃H, -NHSO₂-C₁-C₆-alkyl, CN, NH₂, -NH-C₁-C₆-Alkyl , -N(C₁-C₆-Alkyl)_{2,} NO₂, -S-C₁-C₆-Alkyl, -SO₃-C₁-C₆-Alkyk, -SO-C₁-C₆-Alkyl, -O(CO)C₁-C₆-Alkyl, -COC₁-C₆-Alkyl, -NHCOC₁-C₆-Alkyl oder Halogen, besonders Fluor, Chlor oder Brom;
- D: ein zweibindiger Rest ausgewählt aus der Gruppe bestehend aus -CH₂-CH₂-, -CH=CH-, 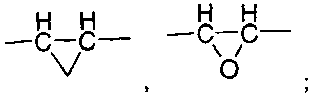
- R⁴: Wasserstoff, C₁-C₆-Alkyl, C₆-C₁₀-Aryl, C₆-C₁₀-Aryl-C₁-C₄-alkylen oder die Gruppe 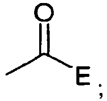
- E: ein Rest ausgewählt aus der Gruppe bestehend aus 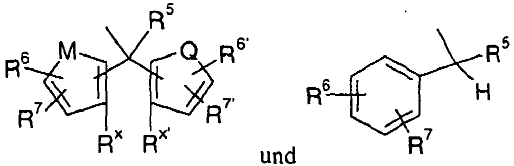
- R⁵: Wasserstoff, Hydroxy, -C₁-C₄-Alkyl, - C₁-C₄-Alkyloxy, - C₁-C₄-Alkylen-Halogen, -O- C₁-C₄-Alkylen-Halogen, - C₁-C₄-Alkylen-OH, -CF₃, CHF₂, -C₁-C₄-Alkylen- C₁-C₄-alkyloxy, -O-CO C₁-C₄-Alkyl, -O-CO C₁-C₄-Alkylen-Halogen, - C₁-C₄-Alkylen-C₃-C₆-Cycloalkyl, -O-COCF₃ oder Halogen;
- M und Q: gleich oder verschieden, bevorzugt gleich, -O-, -S-, -NH-, -CH₂-, -CH=CH-, oder -N(C₁-C₄-Alkyl)-;
- R⁶, R⁷, R^{6'} und R^{7'},: gleich oder verschieden, Wasserstoff, C₁-C₄-Alkyl, C₁-C₄-Alkyloxy, Hydroxy, -CF₃, -CHF₂, CN, NO₂ oder Halogen;
- R^{x} und R^{x'}: gleich oder verschieden Wasserstoff, C₁-C₄-Alkyl, C₁-C₄-Alkyloxy, Hydroxy, -CF₃, -CHF₂, CN, NO₂ oder Halogen
oder
R^{x} und R^{x'} gemeinsam eine Einfachbindung oder eine verbrückende Gruppe ausgewählt aus den Brücken -O-, -S-, -NH-, -CH₂-, -CH₂-CH₂-, -N(C₁-C₄-Alkyl)-, -CH(C₁-C₄-Alkyl)- und -C(C₁-C₄-Alkyl)₂-,
bedeuten, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind Verbindungen der allgemeinen Formel **1**, worin
- n: 1 oder 2;
- A: O, N oder eine Einfachbindung;
- B: ein zweibindiger Rest ausgewählt aus der Gruppe bestehend aus -CH₂-CH₂-, -CH=CH- und -CH₂-O-;
- L: ein zweibindiger Rest ausgewählt aus der Gruppe bestehend aus -CO-(CH₂)ₘ-, -CO-(CH₂)ₘ-NH-CO-(CH₂)ₒ-, -CO-(CH₂)ₘ-CO-NH-(CH₂)ₒ-, -Co-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ;
- m, o: unabhängig voneinander 1 bis 10;
- p, r: unabhängig voneinander 2 bis 10;
- R¹, R^{2,} R³: gleich oder verschieden, Wasserstoff, C₁-C₄-Alkyl, Halogen-C₁-C₄-alkylen, OH, -CF₃, CHF₂, HO-C₁₋₄-alkylen, -O-C₁-C₄-Alkyl, C₆-C₁₀-Aryl, C₆-C₁₀-Aryl-C₁-C₂-alkylen, C₆-C₁₀-Aryl-C₁-C₄-alkylen-O-, -COOH, -COOC₁-C₄-alkyl, -O-C₁-C₄-alkylen-COOH, -O-C₁-C₄-alkylen-COOC₁-C₄-alkyl, -NHSO₃H, -NHSO₂-C₁-C₄-alkyl, CN, NH₂, -NH-C₁-C₄-Alkyl, -N(C₁-C₄-Alkyl)_{2,} NO₂, -S-C₁-C₄-Alkyl, -SO₂-C₁-C₄-Alkyl, -SO-C₁-C₄-Alkyl, -O(CO)C₁-C₄-Alkyl, -COC₁-C₄-Alkyl, -NHCOC₁-C₄-Alkyl oder Halogen, besonders Fluor, Chlor oder Brom;
- D: ein zweibindiger Rest ausgewählt aus der Gruppe bestehend aus -CH₂-CH₂-, -CH=CH-, 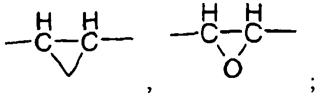
- R⁴: Wasserstoff, C₁-C₄-Alkyl, C₆-C₁₀-Aryl, C₆-C₁₀-Aryl-C₁-C₂-alkylen oder die Gruppe 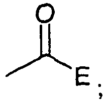
- E: ein Rest ausgewählt aus der Gruppe bestehend aus 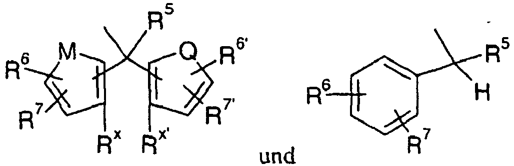
- R⁵: Wasserstoff, Hydroxy, -C₁-C₄-Alkyl, -C₁-C₄-Alkyloxy, -CF₃, -CHF₂, HO-CH₂-, HO-CH₂-CH₂-, Fluor, Chlor oder Brom;
- M und Q: gleich oder verschieden, bevorzugt gleich, -O-, -S-, -NH- oder -CH=CH-;
- R⁶, R⁷, R^{6'} und R^{7'},: gleich oder verschieden, Wasserstoff, C₁-C₄-Alkyl, C₁-C₄-Alkyloxy, Hydroxy, -CF₃, -CHF₂, CN, NO₂, Fluor, Chlor oder Brom;
- R^{x} und R^{x'}: gleich oder verschieden, Wasserstoff, C₁-C₄-Alkyl, C₁-C₄-Alkyloxy, Hydroxy, -CF₃, -CHF₂, CN, NO₂, Fluor, Chlor oder Brom
oder
R^{x} und R^{x'} gemeinsam eine Einfachbindung oder eine verbrückende Gruppe ausgewählt aus den Brücken -O-, -S-, -NH- und -CH₂-,
bedeuten, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin
- n: 1 oder 2, bevorzugt 1;
- A: O, N oder eine Einfachbindung;
- B: ein zweibindiger Rest ausgewählt aus der Gruppe bestehend aus -CH₂-CH₂-, -CH=CH- und -CH₂-O-;
- L: ein zweibindiger Rest ausgewählt aus der Gruppe bestehend aus -CO-(CH₂)ₘ-, -CO-(CH₂)ₘ-NH-CO-(CH₂)ₒ-, -CO-(CH₂)ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ-
- m, o: unabhängig voneinander 1 bis 8;
- p, r: unabhängig voneinander 2 bis 8;
- R¹, R^{2,} R³: gleich oder verschieden, Wasserstoff, C₁-C₄-Alkyl, C₁-C₄-Alkyloxy, Hydroxy, -CF₃, -CHF₂, Phenyl, Benzyl, -COOH, -COOMethyl, -O-CH₂-COOH, -O-CH₂-COOMethyl, -O-CH₂-COOEthyl, -O-CH₂-CH₂-COOH, -O-CH₂-CH₂-COOMethyl, -O-CH₂-CH₂-COOEthyl, CN, NO₂, Fluor, Chlor oder Brom;
- D: ein zweibindiger Rest ausgewählt aus der Gruppe bestehend aus -CH₂-CH₂-, -CH=CH-, 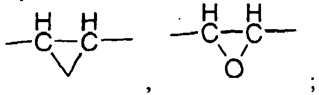
- R⁴: Wasserstoff, Methyl, Ethyl, Phenyl, Benzyl oder die Gruppe 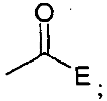
- E: ein Rest ausgewählt aus der Gruppe bestehend aus 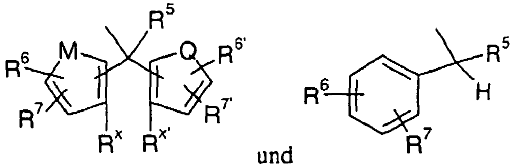
- R⁵: Wasserstoff, Hydroxy, Methyl, Ethyl, Methyloxy, Ethyloxy, -CF₃, HO-CH₂-, HO-CH₂-CH₂- oder Fluor;
- M und Q: gleich oder verschieden, bevorzugt gleich, -S- oder -CH=CH-;
- R⁶, R⁷, R^{6'} und R^{7'},: gleich oder verschieden, Wasserstoff, Methyl, Methyloxy, -CF₃ oder Fluor;
- R^{x} und R^{x'}: gleich oder verschieden Wasserstoff, Methyl, Methyloxy, -CF₃ oder Fluor
oder
R^{x} und R^{x'} gemeinsam eine Einfachbindung oder die verbrückende Gruppe -O-,
bedeuten, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin n 1 bedeutet und worin A, B, D, L, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin A für O (Sauerstoff) öder N (Stickstoff), bevorzugt für N steht und worin n, B, D, L, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin B für -CH=CH- oder -CH₂-O-, bevorzugt für -CH₂-O- steht und worin n, A, D, L, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Besonders bevorzugt sind bei denjenigen Verbindungen, bei denen B für -CH₂-O- steht, die Regioisomere der allgemeinen Formel **1a** 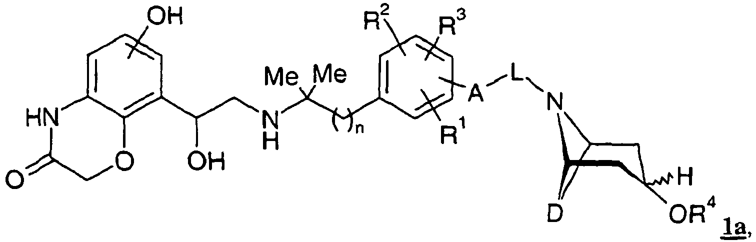 worin n, A, D, L, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit phannakologisch unbedenklichen Säuren sowie gegebenenfalls in Form der Solvate und/oder Hydrate.

Bevorzugt sind ferner Verbindungen der allgemeinen Formel **1**, worin
- L: ein zweibindiger Rest ausgewählt aus der Gruppe bestehend aus -CO-(CH₂)₁₋₆-, -CO-(CH₂)₁₋₆-NH-CO-(CH₂)₁₋₄-, -CO-(CH₂)₁₋₆-CO-NH-(CH₂)₁₋₄-, -CO-(CH₂)₁₋₆-O-(CH₂)₂₋₆-, -(CH₂)₂₋₆-, -(CH₂)₂₋₆-O-(CH₂)₂₋₆-, -(CH₂)₂₋₆-NH-CO-(CH₂)₁₋₄- bedeutet
und worin n, A, B, D, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin
- L: ein zweibindiger Rest ausgewählt aus der Gruppe bestehend aus -CO-(CH₂)₁₋₆-, -CO-(CH₂)₁₋₄-NH-CO-(CH₂)₁₋₂-, -CO-(CH₂)₁₋₄-CO-NH-(CH₂)₁₋₂-, -CO-(CH₂)₁₋₄-O-(CH₂)₂₋₄-, -(CH₂)₂₋₄-, -(CH₂)₂₋₄-O-(CH₂)₂₋₄-, -(CH₂)₂₋₄-NH-CO-(CH₂)₁₋₂- bedeutet
und worin n, A, B, D, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin
- L: ein zweibindiger Rest ausgewählt aus der Gruppe bestehend aus -CO-CH₂-NH-CO-CH₂-, -CO-CH₂-NH-CO-CH₂-CH₂-, -CO-CH₂-CH₂-NH-CO-CH₂-, -CO-CH₂-CH₂-NH-CO-CH₂-CH₂-, -CO-CH₂-CH₂-CH₂-NH-CO-CH₂-, -CO-CH₂-CH₂-CH₂-NH-CO-CH₂-CH₂-, -CO-CH₂-CH₂-CH₂-CH₂-NH-CO-CH₂-, -CO-CH₂-CH₂-CH₂-CH_{Z}-NH-CO-CH₂-CH₂-, -CO-CH₂-, -CO-CH₂-CH₂-, -CO-CH₂-CH₂-CH₂-, -CO-CH₂-CH₂-CH₂-CH₂-, -CO-CH₂-CH₂-CH₂-CH₂-CH₂-, -CO-CH₂-CH₂-CH₂-CH₂-CH₂-CH₂--CO-CH₂-CO-NH-CH₂-, -CO-CH₂-CO-NH-CH₂-CH₂-, -CO-CH₂-CH₂-CO-NH-CH₂-, -CO-CH₂-CH₂-CO-NH-CH₂-CH₂-, -CO-CH₂-CH₂-CH₂-CO-NH-CH₂-, -CO-CH₂-CH₂-CH₂-CO-NH-CH₂-CH₂-, -CO-CH₂-CH₂-CH₂-CH₂-CO-NH-CH₂- und -CO-CH₂-CH₂-CH₂-CH₂-CO-NH-CH₂-CH₂- bedeutet
und worin n, A, B, D, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin
- L: ein zweibindiger Rest ausgewählt aus der Gruppe bestehend aus -CO-CH₂-CH₂-NH-CO-CH₂-, -CO-CH₂-CH₂-NH-CO-CH₂-CH₂-, -CO-CH₂-CH₂-CH₂-NH-CO-CH₂-, -CO-CH₂-CH₂-CH₂-NH-CO-CH₂-CH₂-, -CO-CH₂-CH₂-CH₂-CH₂-NH-CO-CH₂- und -CO-CH₂-CH₂-CH₂-CH₂-NH-CO-CH₂-CH₂- bedeutet
und worin n, A, B, D, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin
- R¹, R^{2,} R³: gleich oder verschieden, Wasserstoff, Methyl, Ethyl, Methoxy, Ethoxy, OH, -CF₃, -CHF₂, Fluor, Chlor oder Brom, bevorzugt Wasserstoff, Methyl, Methoxy, -CF₃, -CHF₂ oder Fluor, besonders bevorzugt Wasserstoff, Methyl, oder Fluor,
bedeuten und worin n, A, B, D, L und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Sol vate und/oder Hydrate.

Bevorzugt sind ferner Verbindungen der allgemeinen Formel **1**, worin
- D: ein zweibindiger Rest ausgewählt aus der Gruppe bestehend aus -CH₂-CH₂-, -CH=CH- und 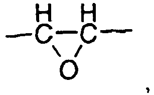 bevorzugt -CH₂-CH₂- oder 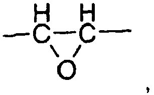 besonders bevorzugt -CH₂-CH₂-
bedeutet und worin n, A, B, L, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin
- D: ein zweibindiger Rest ausgewählt aus der Gruppe bestehend aus -CH₂-CH₂-, -CH=CH- und 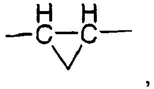 bevorzugt -CH₂-CH₂- oder 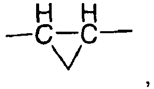 besonders bevorzugt 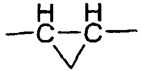
bedeutet und worin n, A, B, L, R¹, R², R³ und R⁴ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin
- R⁴: Wasserstoff, Methyl, Ethyl, Phenyl oder Benzyl, bevorzugt, Wasserstoff, Methyl oder Benzyl, besonders bevorzugt Wasserstoff
bedeutet und worin n, A, B, D, L, R¹, R² und R³ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin
- R⁴: für die Gruppe 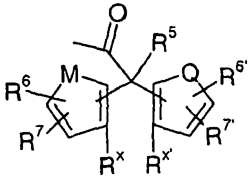 steht,
worin
- R⁵: Wasserstoff, Hydroxy, Methyl oder Fluor, bevorzugt, Wasserstoff, Hydroxy oder Methyl, besonders bevorzugt Hydroxy;
- M und Q: gleich oder verschieden, bevorzugt gleich, -S- oder -CH=CH-;
- R⁶, R⁷, R^{6'} und R^{7'},: gleich oder verschieden, Wasserstoff, -CF₃ oder Fluor, bevorzugt Wasserstoff;
- R^{x} und R^{x'}: gleich oder verschieden Wasserstoff, -CF₃ oder Fluor, bevorzugt Wasserstoff
oder
R^{x} und R^{x'} gemeinsam eine Einfachbindung oder die verbrückende Gruppe -O-
bedeuten und worin n, A, B, D, L, R¹, R² und R³ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Bevorzugt sind ferner Verbindungen der allgemeinen Formel **1**, worin
- R⁴: für die Gruppe 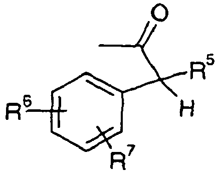 steht,
worin
- R⁵: Wasserstoff, Hydroxy, Methyl, -CF₃ oder HO-CH₂-;
- R⁶ und R⁷: gleich oder verschieden, Wasserstoff, Methyl, Methyloxy, -CF₃ oder Fluor, bevorzugt Wasserstoff oder Fluor, besonders bevorzugt Wasserstoff;
bedeuten und worin n, A, B, D, L, R¹, R² und R³ die vor- oder nachstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Ein weiterer Aspekt der vorliegenden Erfindung betrifft die vorstehend genannten neuen Verbindungen der Formel **1** in Form der einzelnen optischen Isomeren, Mischungen der einzelnen Enantiomere oder gegebenenfalls Diastereomeren oder Racemate. Besonders bevorzugt sind dabei Verbindungen der Formel **1** in Form der enantiomeren- oder diastereomerenreinen Verbindungen, wobei diejenigen R-Enantiomere oder gegebenenfalls Diastereomere der Verbindungen der Formel **1** erfindungsgemäß von herausragender Bedeutung sind, die durch die allgemeine Formel ***R*-1** darstellbar sind, 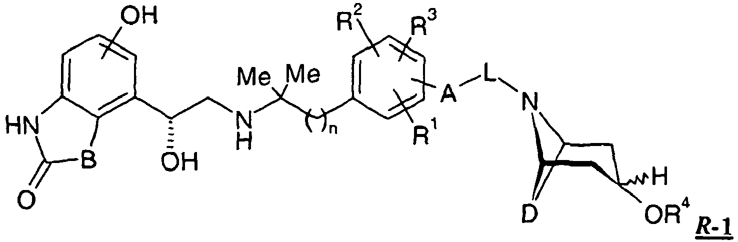 worin n, A, B, D, L, R¹, R², R³ und R⁴ die vorstehend genannten Bedeutungen haben können, gegebenenfalls in Form ihrer Säureadditionssalze mit pharmakologisch unbedenklichen Säuren sowie gegebenenfalls in Form der Solvate und/oder Hydrate.

Die Verbindungen der allgemeinen Formel **1** können hinsichtlich der Stellung der Hydroxylfunktion in 3 verschiedenen Regioisomeren vorliegen.

Erfindungsgemäß bevorzugte Regioisomere der Verbindungen der allgemeinen Formel **1** sind jene der allgemeinen Formel ***Regio*-1a** 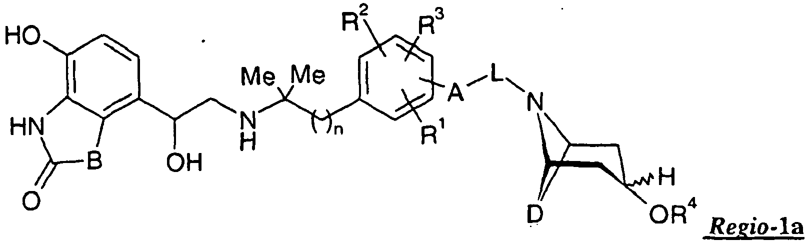 worin n, A, B, D, L, R¹, R², R³ und R⁴ die vorstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Erfindungsgemäß ferner bevorzugte Regioisomere der Verbindungen der allgemeinen Formel **1** sind jene der allgemeinen Formel ***Regio*-1b** 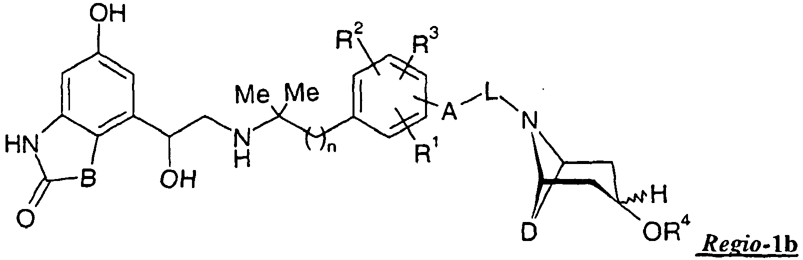 worin n, A, B, D, L, R¹, R², R³ und R⁴ die vorstehend genannten Bedeutungen haben können, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

Als Alkylgruppen werden, soweit nicht anders angegeben, verzweigte und unverzweigte Alkylgruppen mit 1 bis 6, bevorzugt mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Alkylengruppen werden, soweit nicht anders angegeben, verzweigte und unverzweigte zweibindige Alkylbrücken mit 1 bis 6, bevorzugt mit 1 bis 4 Kohlenstoffatomen bezeichnet. Beispielsweise werden genannt: Methylen, Ethylen, n-Propylen oder n-Butylen.

Als Alkylen-Halogen-Gruppen werden, soweit nicht anders angegeben, verzweigte und unverzweigte zweibindige Alkylbrücken mit 1 bis 6, bevorzugt mit 1 bis 4 Kohlenstoffatomen bezeichnet, die ein-, zwei- oder dreifach, bevorzugt zweifach durch ein Halogen substituiert sind. Dementsprechend werden als Alkylen-OH-Gruppen, soweit nicht anders angegeben, verzweigte und unverzweigte zweibindige Alkylbrücken mit 1 bis 6, bevorzugt mit 1 bis 4 Kohlenstoffatomen bezeichnet, die ein-, zwei- oder dreifach, bevorzugt einfach durch ein Hydroxy substituiert sind.

Als Alkyloxygruppen (oder auch -O-Alkylgruppen oder Alkoxygruppen) werden, soweit nicht anders angegeben, verzweigte und unverzweigte Alkylgruppen mit 1 bis 6, bevorzugt mit 1 bis 4 Kohlenstoffatomen bezeichnet, die über ein Sauerstoffatom verknüpft sind. Beispielsweise werden genannt: Methylox, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Der Begriff C₆-C₁₀-Aryl steht für ein aromatisches Ringsystem mit 6 bis 10 Kohlenstoffatomen. Bevorzugte Arylreste sind Phenyl oder Naphthyl.

Der Begriff C₆-C₁₀-Aryl-C₁-C₄-alkylen steht für ein aromatisches Ringsystem mit 6 bis 10 Kohlenstoffatomen, welches über eine Alkylenbrücke mit 1 bis 4 C-Atomen verknüpft ist. Bevorzugte Aralkylenreste sind Benzyl und Phenylethyl.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Ein weiterer Aspekt der vorliegenden Erfindung betrifft die vorstehend genannten Verbindungen der Formel **1** in Form ihrer Säureadditionssalze mit pharmakologisch unbedenklichen Säuren sowie gegebenenfalls in Form der Solvate und/oder Hydrate.

Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Die erfindungsgemäßen Verbindungen der Formel 1 zeichnen sich durch ihre antagonistische Wirkung an muskarinischen Rezeptoren vom Subtyp M3 und durch ihre agonistische Wirkung am adrenergen β2-Rezeptor aus. Die erfindungsgemäßen Verbindungen weisen bezüglich der Affinität zum M3-Rezeptor Ki-Werte von kleiner 1 µM und bezüglich der β2-agonistischen Wirkung EC50-Werte von kleiner 1 µM auf. Diese Werte wurden entsprechend der nachfolgend beschriebenen Vorgehensweise bestimmt.

### M3-Rezeptorbindung:

Für den Bindungsassay wurden Membranpräparationen von CHO Zellen (Chinese hamster ovary) verwendet, die mit dem entsprechenden Gen des humanen muskarinischen Rezeptorsubtyps hm3 transfiziert sind. Der Test wurde in einem Endvolumen von 1 ml ausgeführt und setzte sich zusammen aus 100 µl unmarkierter Substanz in verschiedenen Konzentrationen, 100 µl Radioligand (3H-N-Methylscopolamin 2 nmol/L (3H-NMS), 200 µl Membranpräparation und 600 µl HEPES Puffer (20 mmol/L HEPES, 10 mmol/L MgCl2, 100 mmol/L NaCl, mit 1 mol/L NaOH auf pH 7.4 eingestellt). Die unspezifische Bindung wurde durch 10 µmol/L Atropin ermittelt.

Die Inkubation erfolgte für 45 min. bei 37°C in 96-well Mikrotiterplatten (Beckman, Polystyrol, Nr. 267001) als Doppelbestimmung. Die Inkubation endete durch Filtration mittels Inotech Zellemter (Typ IH 110) über Whatman G-7 Filter. Die Filter wurden mit 3 ml eisgekühltem HEPES Puffer gewaschen und vor der Messung getrocknet. Die Radioaktivität der Fittermatten wurden simultan mittels zweidimensionalem, digitalem Autoradiograph (Berthold, Wildbad, Typ 3052) gemessen. Die Ki-Werte wurden mit impliziten Gleichungen, die direkt vom Massenwirkungsgesetz abgeleitet wurden, mit dem Modell für die 1 Rezeptor 2 Liganden Reaktion (SysFit - Software, SCHITTKOWSKI) berechnet.

### Literatur:

BONNER TI: New subtypes of muscarinic acetylcholine receptors Trends Pharmacol. Sci. 10, Suppl.: 11-15 (1989)
SCHITTKOWSKI K: Parameter estimation in systems of nonlinear equations Numer Math. 68: 129-142 (1994).

### β2-agonistische Wirkung:

Die Bestimmung der agonistischen Wirkung am ß₂ Rezeptor erfolgte über die Messung der cAMP Synthese in CHO (Chinese hamster ovary) Zellen, die den humanen ß₂ adrenergen Rezeptor stabil exprimierten.

Die Zellen wurden nachdem sie 70-80% konfluent gewachsen waren, 3 x mit PBS gewaschen, von der Platte gelöst und in Stimulationspuffer (0.1%BSA, 0.5 mM IBMX, 5 mM Hepes, 1x HBSS) mit einer Zellzahl von 0.65 x 10⁶ Zellen/ml suspendiert. Anschließend wurden 20 µl der Zellsuspension mit 10 µl Testsubstanz (in 0.9% NaCl, 3% DMSO) inkubiert (finale Konzentration der Testsubstanz von 0,01 nM bis 10 µM). Nach einer Inkubation für 30 min bei Raumtemperatur wurden die Zellen durch Zugabe von 20 µl Lysispuffer (0.3% Tween 20, 5 mM Hepes, 0.1% BSA) für mindestens 30 min auf Eis lysiert. Im Anschluss daran wurden 5 µl des Lysates in 4-fach Bestimmung auf eine 384 Well Platte (OptiPlate; Packard Bioscience) pipettiert. Die Detektion des synthetisierten cAMP erfolgte mittels des AlphaScreen cAMP Detection Kits der Firma BioSignal Packard und anschließender Messung an einem Fusion Reader (Packard Bioscience).

Die resultierenden Alpha Screen Counts (ACS) wurden über einen cAMP Standardkurve direkt als cAMP Synthese umgerechnet. Zur Bestimmung des Stimulationslevels ("intrinsische Aktivität") wurden auf jeder Testplatte Zellen nur mit Kontrolllösung (0.9% NaCl, 3% DMSO) versetzt (= nicht stimulierte Zellen, "low") bzw. mit dem vollen ß Rezeptor Agonisten Isoprenalin (1 nM bis 10 µM; = zu 100% stimulierte Zellen; "high"). Zur Analyse der Daten wurde die maximale cAMP Synthese der jeweiligen Testsubstanz mit der des vollen ß Agonisten Isoprenalin nach folgender Formel errechnet:
[cAMP(Substanz)] - [cAMP(low)] X 100/[cAMP(high)-[cAMP(low)].

Die Bestimmung der ED50 erfolgte mit der Software Graph Pad Prism.

Die erfindungsgemäßen Verbindungen der Formel **1** zeichnen sich durch vielfältige Anwendungsmöglichkeiten auf therapeutischem Gebiet aus. Hervorzuheben sind erfindungsgemäß solche Anwendungsmöglichkeiten, für welche die erfindungsgemäßen Verbindungen der Formel **1** aufgrund ihrer pharmazeutischen Wirksamkeit als Anticholinergikum und Betamimetikum bevorzugt zur Anwendung gelangen können.

Ein weiterer Aspekt der vorliegenden Erfindung betrifft dementsprechend die vorstehend genannten neuen Verbindungen der Formel **1** als Arzneimittel. Die vorliegende Erfindung betrifft ferner die Verwendung der vorstehend genannten Verbindungen der allgemeinen Formel **1** zur Herstellung eines Arzneimittels zur Behandlung von Atemwegserkrankungen.

Die vorliegende Erfindung betrifft bevorzugt die Verwendung der vorstehend genannten Verbindungen der allgemeinen Formel **1** zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die Verwendung von Verbindungen der allgemeinen Formel **1** zur Herstellung eines Arzneimittels zur Behandlung von Obstruktive Lungenerkrankungen die ausgewählt sind aus der Gruppe bestehend aus COPD (chronisch obstruktive pulmonale Erkrankung), Asthma Bronchiale, pädiatrisches Asthma, schweres Asthma, akuter Asthma-Anfall und chronische Bronchitis, wobei die Verwendung zur Herstellung eines Arzneimittels zur Behandlung von Asthma Bronchiale erfindungsgemäß besonders bevorzugt ist.

Bevorzugt ist ferner die Verwendung von Verbindungen der allgemeinen Formel **1** zur Herstellung eines Arzneimittels zur Behandlung von Lungenemphysemen die ihren Ursprung haben in COPD (chronisch obstruktive pulmonale Erkrankung) oder α1-Proteinase-Inhibitor-Mangel.

Bevorzugt ist ferner die Verwendung von Verbindungen der allgemeinen Formel **1** zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die Verwendung von Verbindungen der allgemeinen Formel **1** zur Herstellung eines Arzneimittels zur Behandlung von Interstitiellen Lungenerkrankungen, die ausgewählt sind aus der Gruppe bestehend aus infektiös bedingte Pneumonien, wie beispislweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung von Verbindungen der allgemeinen Formel **1** zur Herstellung eines Arzneimittels zur Behandlung von Zystischer Fibrose bzw. Mukoviszidose.

Bevorzugt ist ferner die Verwendung von Verbindungen der allgemeinen Formel **1** zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung von Verbindungen der allgemeinen Formel **1** zur Herstellung eines Arzneimittels zur Behandlung von Bronchiektasen.

Bevorzugt ist ferner die Verwendung von Verbindungen der allgemeinen Formel **1** zur Herstellung eines Arzneimittels zur Behandlung von ARDS (adult respiratory distress syndrom).

Bevorzugt ist ferner die Verwendung von Verbindungen der allgemeinen Formel **1** zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der Verbindungen der Formel **1** zur Herstellung eines Arzneimittels zur Behandlung von Asthma oder COPD. Von besonderer Bedeutung ist ferner die vorstehend genannte Verwendung von Verbindungen der Formel **1** zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Ferner betrifft die vorliegende Erfindung ein Verfahren zur Behandlung der vorstehend genannten Erkrankungen, dadurch gekennzeichnet, daß eine oder mehrere der vorstehend genannten Verbindungen der allgemeinen Formel **1** in therapeutisch wirksamen Mengen appliziert werden. Die vorliegende Erfindung betrifft bevorzugt Verfahren zur Behandlung von Asthma oder COPD, dadurch gekennzeichnet, daß eine oder mehrere der vorstehend genannten Verbindungen der allgemeinen Formel **1** in therapeutisch wirksamen Mengen einmal täglich appliziert werden.

Die erfindungsgemäßen Verbindungen der Formel **1** zeichnen sich durch eine lange Wirkungsdauer nach inhalativer Gabe aus. Dies konnte durch die nachfolgende Untersuchung belegt werden:

Im narkotisierten Hund kann durch intravenöse Gaben von Acetylcholin (ACh) eine Bronchokonstriktion induziert werden. Inhalative Bronchodilatoren, beispielsweise Anticholinergika oder beta-2-Mimetika schützen die Tiere vor einem solchen AChinduzieren Bronchospasmus. In der Studie wurde bei mit Propofol anästhesierten, künstlich beatmeten Beagle Hunden eine Vene, z.B. Ateria cephalica, für die intravenöse ACh Gaben vorbereitet. Während der Narkose werden die Vitalparameter der Tiere fortlaufend gemessen, die physiologische Körpertemperatur wird durch Heizsysteme konstant gehalten. Der Transpulmonale Druck wurde mit einem Differenzialdruckmesser gemessen.

Ein Fleisch'sches Pneumotachometer ist verbunden mit dem Tracheotubus für die Messung des Atemflusses. EKG und diastolischer/systolischer Blutdruck werden zusätzlich gemessen.

Nach einer Eingewöhnungsphase werden den Tieren die Testsubstanzen inhalativ mit dem Respimat appliziert. ACh wird 30 und 15 min vor, sowie 5, 10, 20, 30, 120 und 180 min nach Inhalation der Testsubstanz appliziert. Nach dem letzten Wert wird die Infusionsnarkose beendet, die Tiere wachen wieder auf. Am folgenden Tag werden die Tiere zur Messung der Bronchoprotektion 24 h nach Substanzgabe erneut in Kurzzeit-Narkose gelegt. Die erhaltenen Daten werden berechnet als % Bronchoprotektion gegenüber den Werten vor Substanzgabe.

Die Verbindungen der allgemeinen Formel **1** lassen sich nach dem im Schema 1 skizzierten Weg darstellen.

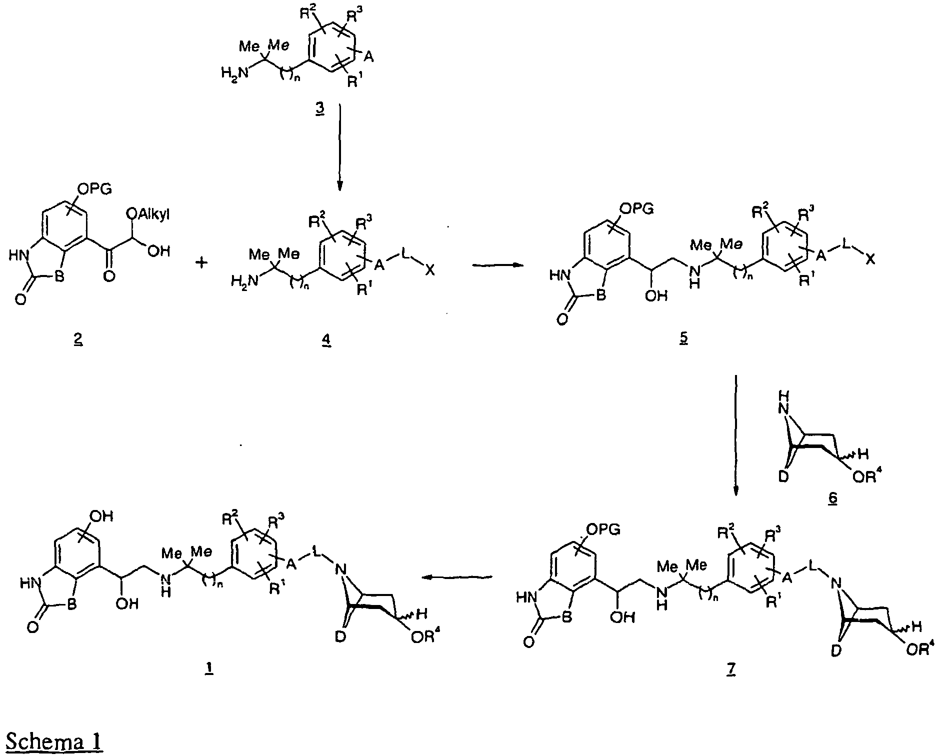

In den in Schema 1 genannten Ausgangsverbindungen und Zwischenprodukten können die Gruppen n, A, B, D, L, R¹, R², R³ und R⁴ die vorstehend genannten Bedeutungen und die Gruppen OPG und X die nachstehend definierten Bedeutungen aufweisen.

Eine Verbindung der allgemeinen Formel **2** wird mit einer Verbindung der allgemeinen Formel **4** in einem Lösungsmittel, vorzugsweise einem Alkohol, besonders bevorzugt Ethanol zur Schiffschen Base kondensiert und anschließen mit einem Reduktionsmittel wie z.B. H₂/Pd/C oder NaBhL₄ zum Aminoalkohol **5** reduziert. Ist X eine geschützte Aminogruppe so wird im nächsten Schritt die Schutzgruppe, vorzugsweise die BOC-Gruppe mit Trifluoressigsäure, gespalten und mit einem ω-Halogencarbonsäurehalogenid acyliert. Anschließend wird mit Verbindung **6** substituiert und man erhält Verbindung **7**. Nach Abspaltung der Schutzgruppe PG, im Fall einer Benzylschutzgruppe durch hydrogenolytischer Spaltung, gelangt man zur Zielverbindungen **1**.

Ist X in Verbindung **5** eine Abgangsgruppe, so kann diese direkt mit Verbindung **6** substituiert werden.

Die Verbindung **4** kann je nach Ausgestaltung von A und L durch Amidbildung, Esterbildung, Aminbildung oder Etherbildung aus Verbindung **3** und einem geeigneten Linker L nach an sich bekannten Verfahren dargestellt werden.

Ein weiterer Aspekt der vorliegenden Erfindung betrifft die Zwischenprodukte der allgemeinen Formel **4** 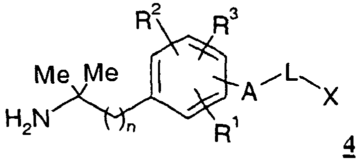 worin
- X: Chlor, Brom, Iod, Methansulfonat, Trifluormethansulfonat, p-Toluolsulfonat, oder eine durch eine geeignete Schutzgruppe geschützte Aminofunktion, vorzugsweise -NH-Boc, -NH-Cbz, -NH-Fmoc, -NH-Teoc, oder -NH-Alloc
bedeuten und worin n, A, L, R¹, R² und R³ die vorstehend genannten Bedeutungen aufweisen können.

In Übereinstimmung mit im Stand der Technik hierfür verwendeten Bezeichnungen, steht dabei -NH-Boc für tert-Butylcarbarnat, -NH-Cbz für Benzylcarbamate, -NH-Fmoc für 9-Fluorenylmethylcarbarnat, -NH-Teoc für 2-Trimethylsilylethylcarbamat, und -NH-Alloc für Allylcarbamat.

Bevorzugt sind Zwischenprodukte der allgemeinen Formel **4**, worin
- X: Chlor, Brom, Iod, Methansulfonat, Trifluormethansulfonat, p-Toluolsulfonat oder -NH-Boc
bedeuten und worin n, A, L, R¹, R² und R³ die vorstehend genannten Bedeutungen aufweisen können.

Ein weiterer Aspekt der vorliegenden Erfindung betrifft die Zwischenprodukte der allgemeinen Formel **5** 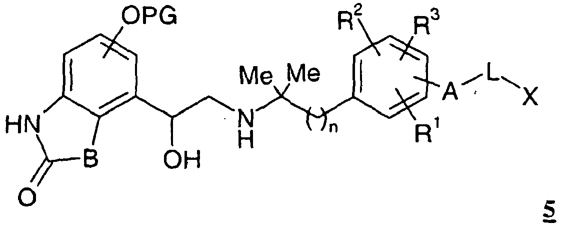 worin
- OPG: eine durch eine Schutzgruppe PG geschützte Hydroxyfunktion, vorzugsweise -O-C₁-C₄-Alkyl, -O-Benzyl oder -O-CH₂-O-C₁-C₄-Alkyl, bevorzugt -O-Methyl, -O-Ethyl, -O-Benzyl oder -O-CH₂-O-Methyl, besonders bevorzugt -O-Ethyl oder -O-Benzyl, besonders bevorzugt -O-Benzyl
bedeutet und worin n, A, L, X, R¹, R² und R³ die vorstehend genannten Bedeutungen aufweisen können.

Ein weiterer Aspekt der vorliegenden Erfindung betrifft die Zwischenprodukte der allgemeinen Formel **7** 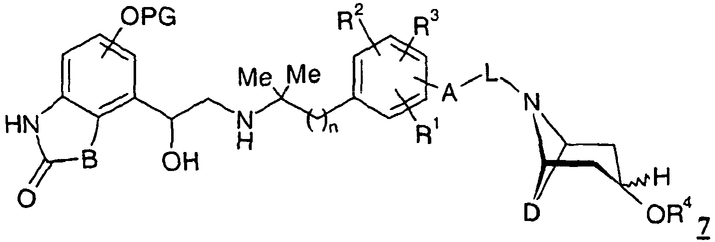 worin n, A, L, D, X, OPG, R¹, R² und R³ die vorstehend genannten Bedeutungen aufweisen können.

Die neuen Verbindungen der allgemeinen Formel **1** können in Analogie der nachfolgenden Beispiele dargestellt werden. Die nachstehend beschriebenen Beispiele sind als Erläuterung der Erfindung zu verstehen ohne diese jedoch einzuschränken.

### Beispiel 1:

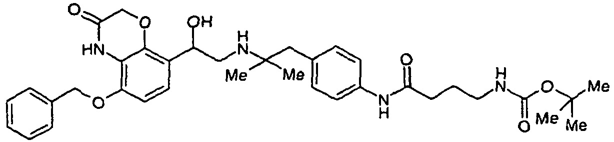

17.00 g (203 mmol) 4-tert-Butoxycarbonylamino-buttersäure in 300 mL THF wird auf -25 °C gekühlt, mit 9.7 mL (101 mmol) N-Methylmorpholin und 11.4 mL (137 mmol) Chlorameisensäureisobutylester versetzt und 30 min bei -25 °C gerührt. 17.5 g (201 mmol) 4-(2-Amino-2-methyl-propyl)-phenylamin und 9.7 mL (101 mmol) N-Methylmorpholin werden zugegeben, das Gemisch langsam auf RT erwärmt und 16 h bei RT gerührt. Das Reaktionsgemisch wird mit ges. wässerige Natriumbicarbonat-Lösung versetzt, die Phasen getrennt und die wässerige Phase mit EtOAc extrahiert. Die vereinigten org. Extrakte werden über Natriumsulfat getrocknet und i. Vak. eingeengt. Der Rückstand wird über Kieselgel (FM: CH₂Cl₂/MeOH/NH₄OH Gradient von 95/4,5/0,5 bis 60/35/5) gereinigt. Die entsprechenden Fraktionen werden vereinigt und i. Vak. eingeengt. Ausbeute 21.5 g (74%) als braunes Öl.

40 g (112 mmol) 5-Benzyloxy-8-(2-ethoxy-2-hydroxyacetyl)-4H-benzo[1,4]oxazin-3-on und 40 g (114 mmol) N-(2-Amino-2-methyl-proyl)-phenyl)-4-(tert-butyloxy-carbonylamino)-butansäureamid werden in 300 mL Ethanol vorgelegt und 30 min auf 80°C erwärmt, dann 30 min bei 50°C gerührt. Anschließend wird abgekühlt, NaBH₄ zugegeben und 2 h bei RT gerührt. Es wird mit etwas Aceton versetzt, 30 min bei RT gerührt, mit Eisessig angesäuert und i. Vak. eingeengt. Der Rückstand wird in Dichlormethan aufgenommen, mit 1M KHSO₄-Lsg und anschließend mit 15%iger K₂CO₃-Lsg. gewaschen. Die org. Phase wird nochmals mit ges. NaHCO₃-Lsg. ausgeschüttelt, über Na₂SO₄ getrocknet und i. Vak. eingeengt. Der Rückstand wird über Kieselgel (FM: CH₂Cl₂/MeOH/NH₄OH Gradient von 95/4,5/0,5 bis 60/35/5) gereinigt. Entsprechende Fraktionen vereinigt und i. Vak. eingeengt. Ausbeute 60,5 g (84%) Öl.

### Beispiel 2:

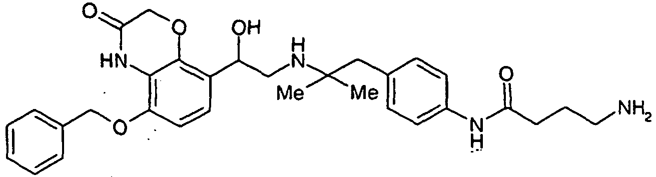

57g (89 mmol) der Verbindung aus Beispiel 1 werden in 800 mL Dichlormethan gelöst, 100 mL Trifluoressigsäure zugegeben und über Nacht bei RT gerührt. Das Lösemittel wird i. Vak. entfernt, der Rückstand in Essigester und Wasser aufgenommen, mit 130 mL 30%iger NaOH alkalisch gestellt und die Phasen getrennt. Die wässrige Phase wird nochmals mit Essigester extrahiert, die org. Extrakte vereinigt und mit ges. NaHCO₃-Lsg. gewaschen, über Na₂SO₄ getrocknet und i. Vak. eingeengt. Der Rückstand wird über Kieselgel (FM: CH₂Cl₂/MeOH/NH₄OH = 90/10/1) gereinigt. Die entsprechende Fraktionen werden vereinigt und i. Vak. eingeengt. Ausbeute: 20,5 g (42%) brauner Feststoff.

### Beispiel 3:

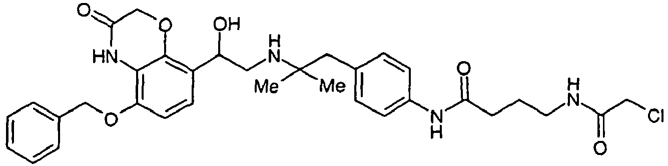

2 g (3,7 mmol) der Verbindung aus Beispiel 2 werden in 10 mL Dichlormethan gelöst, 0,65 mL (3,7 mmol) Hünigbase zugegeben und im Eisbad gekühlt. 0,3 mL (3,7 mmol) Chloracetylchorid werden in 10 mL Dichlormethan gelöst und langsam zugetropft. Es wird 30 min bei Kühlung nachgerührt, dann das Eisbad entfernt und 2 h bei RT nachgerührt. Das Reaktionsgemisch wird mit etwas Dichlormethan verdünnt und mit 15 %iger K₂CO₃-Lsg. gewaschen. Die org. Phase wird über Na₂SO₄ getrocknet und i. Vak. eingeengt. Der Rückstand wird über Kieselgel (FM: CH₂Cl₂/MeOH/NH₄OH Gradient von 95/4,5/0,5 bis 60/35/5) gereinigt. Die entsprechende Fraktionen werden vereinigt und i. Vak. eingeengt. Ausbeute: 2,1 g (92%) Öl.

### Beispiel 4:

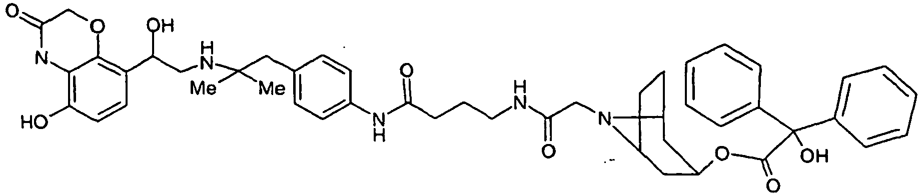

470 mg (0,7 mmol) der Verbindung aus Beispiel 3 werden in 10 mL DMF gelöst, 300 mg (0,8 mmol) Benzylsäure-nortropanylester, 370 mg K₂CO₃ zugegeben und über Nacht bei 100 °C gerührt. Das Lösemittel wird i. Vak. abgezogen, der Rückstand in Wasser aufgenommen und zweimal mit Essigester extrahiert. Die org. Extrakte werden vereinigt, über Na₂SO₄ getrocknet und i. Vak. eingeengt. Der Rückstand wird über Kieselgel (FM: CH₂Cl₂/MeOH/NH₄OH Gradient von 95/4,5/0,5 bis 60/35/5) gereinigt. Die entsprechende Fraktionen werden vereinigt und i. Vak. eingeengt. Der Rückstand wird in Diisopropylether verrieben und abgesaugt. Anschließend wird in 20 mL Methanol gelöst, an 50 mg Pd/C (10%) und 3 bar Wasserstoff 2 h bei RT geschüttelt. Der Kat. wird abgesaugt, das Lösemittel i. Vak. eingeengt, der Rückstand in Diisopropylether verrieben und abgesaugt. Ausbeute: 102 mg (16%); Massenspektroskopie: [M+H] = 834.

### Beispiel 5:

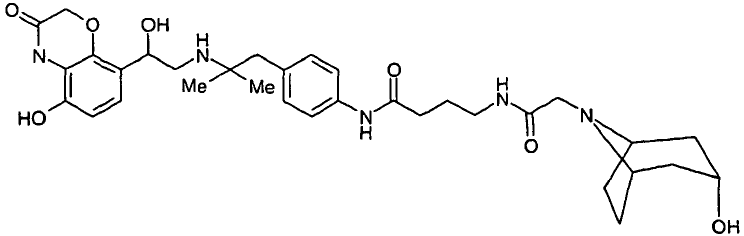

100 mg (0,16 mmol) der Verbindung aus Beispiel 3 werden in THF gelöst, 25 mg (0,20 mmol) Nortropanol und 0,035 mL (0,20 mmol) Hünigbase zugegeben. Es wird 72 h bei 60°C gerührt, das Lösemittel i. Vak. abgezogen, der Rückstand in Dichlormethan aufgenommen und mit ges. NaHCO₃-Lsg. gewaschen. Die org. Phase wird über Na₂SO₄ getrocknet und i. Vak. eingeengt. Der Rückstand wird über Kieselgel (FM: CH₂Cl₂/MeOH/NH₄OH Gradient von 95/4,5/0,5 bis 60/35/5) gereinigt. Die entsprechende Fraktionen vereinigt und i. Vak. eingeengt. Der Rückstand wird in Diisopropylether verrieben und abgesaugt. Anschließend wird in 10 mL Methanol gelöst, an 40 mg Pd/C (10%) und 3 bar Wasserstoff 2 h bei RT geschüttelt. Der Kat. wird abgesaugt, das Lösemittel i. Vak. eingeengt, der Rückstand in Diisopropylether verrieben und abgesaugt. Ausbeute: 64 mg (64%); Massenspektroskopie: [M+H] = 624.

### Beispiel 6:

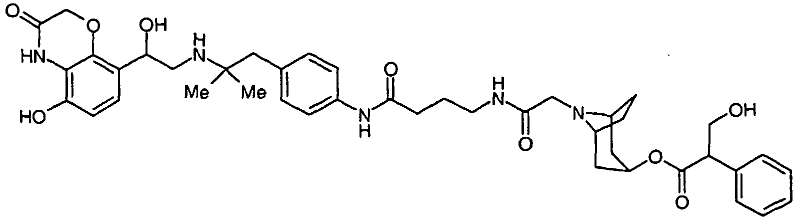

470 mg (0,75 mmol) der Verbindung aus Beispiel 3 werden in 100 mL DMF gelöst, 210 mg (0,76 mmol) Tropasäure-nortropanylester und 370 mg K₂CO₃ zugegeben und über Nacht bei RT gerührt. Das Lösemittel wird i. Vak. abgezogen, der Rückstand in 15%iger KzCO₃-Lsg aufgenommen und zweimal mit Dichlormethan extrahiert. Die org. Extrakte werden vereinigt, über Na₂SO₄ getrocknet und i. Vak. eingeengt. Der Rückstand wird über Kieselgel (FM: CH₂Cl₂/MeOH/NH₄OH Gradient von 95/4,5/0,5 bis 60/35/5) gereinigt. Die entsprechende Fraktionen werden vereinigt, i. Vak. eingeengt, Rückstand in Diisopropylether verrieben und abgesaugt. Anschließend wird in 15 mL Methanol gelöst und an 50 mg Pd/C (10%) und 3 bar Wasserstoff 1 h bei RT geschüttelt. Der Kat. wird abgesaugt, das Lösemittel i. Vak. eingeengt, der Rückstand in Diisopropylether verrieben und abgesaugt. Ausbeute: 85 mg (16%); Massenspektroskopie: [M+H] = 772.

Geeignete Anwendungsformen zur Applikation der Verbindungen der Formel **1** sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilfslösungsmittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserem oder Farbstoffen versetzt werden.

Bei der erfindungsgemäßen Applikation der Verbindungen der Formel **1** zur Therapie von Atemwegeserkrankungen werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erindungsgemäß einsetzbare Inhalationspulver können **1** entweder allein oder im Gemisch mit geeigneten physiologisch unbedenkliche Hilfsstoffen enthalten.

Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure und Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.

Den treibgasfreien Inhaltionslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniurnchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/ 100ml, besonders bevorzugt zwischen 5 und 20 mg/100ml enthalten.

Bevorzugte Formulierungen enthalten außer dem Lösungsmittel Wasser und dem Wirkstoff **1** nur noch Benzalkoniumchlorid und Natriumedetat.

In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen.

Ein weiterer Aspekt der vorliegenden Erfindung betrifft die vorstehend genannten pharmazeutischen Formulierungen, gekennzeichnet durch einen Gehalt einer Verbindung der Formel **1**, als solche, besonders bevorzugt die vorstehend genannten inhalativ applizierbaren pharmazeutischen Formulierungen.

Die nachfolgenden Formulierungsbeipiele illustrieren die vorliegende Erfindung ohne sie jedoch in ihrem Umfang zu beschränken:

| A) | Ampullenlösung | |
|---|---|---|
| | Wirkstoff der Formel **1** | 25 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| B) | Dosieraerosol (Suspension) | |
|---|---|---|
| | Wirkstoff der Formel **1** | 0.03 Gew.% |
| | Sorbitantrioleat | 0.6 Gew. *%* |
| | TG134a : TG227 2:1 | 99.37 Gew.% |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden

| C) | Dosieraerosol (Lösung) | |
|---|---|---|
| | Wirkstoff der Formel **1** | 0.03 Gew.% |
| | Ethanol abs. | 20 Gew.% |
| | wässrige HCl 0.01 mol/l | 2.0 Gew.% |
| | TG134a | 77.97 Gew.% |

Die Herstellung der Lösung erfolgt in üblicher Art und Weise durch Mischen der einzelnen Bestandteile.

| D) | Inhalationspulver | |
|---|---|---|
| | Wirkstoff der Formel **1** | 80 *µ*g |
| | Lactose Monohydrat ad | 10 mg |

Die Herstellung des Inhalationspulvers erfolgt in üblicher Art und Weise durch Mischen der einzelnen Bestandteile.

## Patentansprüche

1. Verbindungen der allgemeinen Formel **1** 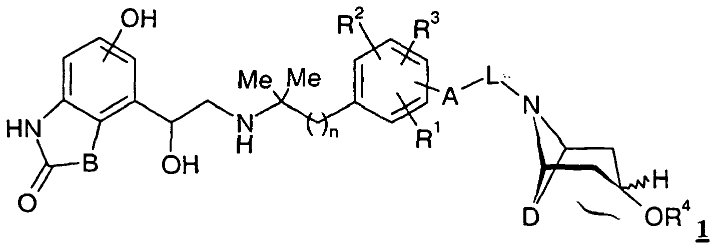 worin
n 1, 2 oder 3, bevorzugt 1 und 2;
A O, N oder eine Einfachbindung;
B ein zweibindiger Rest ausgewählt aus der Gruppe bestehend aus -CH₂-CH₂-, -CH=CH- und -CH₂-O-;
L ein zweibindiger Rest ausgewählt aus der Gruppe bestehend aus -CO-(CH₂)ₘ-, -CO-(CH₂)ₘ-NH-CO-(CH₂)ₒ-, -CO-(CH₂)ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ-, -(CH₂)ₚ-CO-NH-(CH₂)ₒ-, SO₂-(CH₂)ₘ-, -SO₂-(CH₂)ₘ-NH-CO-(CH₂)ₒ-, -SO₂-(CH₂)ₘ-CO-NH-(CH₂)ₒ-, -SO₂-(CH₂)ₘ-O-(CH₂)ₚ-;
m, o unabhängig voneinander 1 bis 12;
p, r unabhängig voneinander 2 bis 12;
R¹, R^{2,} R³ gleich oder verschieden, Wasserstoff, C₁-C₆-Alkyl, Halogen-C₁-C₆-alkylen, OH, -CF₃, CHF₂, HO-C₁₋₆-alkylen, -O-C₁-C₆-Alkyl, C₆-C₁₀-Aryl, C₆-C₁₀-Aryl-C₁-C₄-alkylen, C₆-C₁₀-Aryl-C₁-C₆-alkylen-O-, -COOH, -COOC₁-C₆-alkyl, -O-C₁-C₆-alkylen-COOH, -O-C₁-C₆-alkylen-COOC₁-C₆-alkyl, -NHSO₃H, -NHSO₂-C₁-C₆-alkyl, CN, NH₂, -NH-C₁-C₆-Alkyl, -N(C₁-C₆-Alkyl)₂, NO₂, -S-C₁-C₆-Alkyl, -SO₂-C₁-C₆-Alkyl, -SO-C₁-C₆-Alkyl, -O(CO)C₁-C₆-Alkyl, -COC₁-C₆-Alkyl, -NHCOC₁-C₆-Alkyl oder Halogen, besonders Fluor, Chlor oder Brom;
D ein zweibindiger Rest ausgewählt aus der Gruppe bestehend aus -CH₂-CH₂-, -CH=CH-, 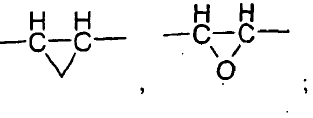
R⁴ Wasserstoff, C₁-C₆-Alkyl, C₆-C₁₀-Aryl, C₆-C₁₀-Aryl-C₁-C₄-alkylen oder die Gruppe 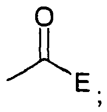
E ein Rest ausgewählt aus der Gruppe bestehend aus 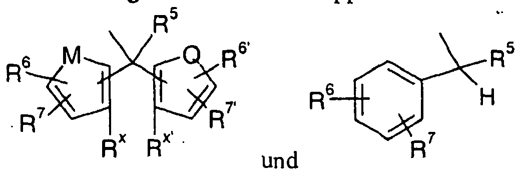
R⁵ Wasserstoff, Hydroxy, -C₁-C₄-Alkyl, - C₁-C₄-Alkyloxy, - C₁-C₄-Alkylen-Halogen, -O- C₁-C₄-Alkylen-Halogen, - C₁-C₄-Alkylen-OH, -CF₃, CHF₂, -C₁-C₄-Alkylen- C₁-C₄-alkyloxy, -O-CO C₁-C₄-Alkyl, -O-CO C₁-C₄-Alkylen-Halogen, - C₁-C₄-Alkylen-C₃-C₆-Cycloalkyl, -O-COCF₃ oder Halogen;
M und Q gleich oder verschieden, bevorzugt gleich, -O-, -S-, -NH-, -CH₂-, -CH=CH-, oder -N(C₁-C₄-Alkyl)-;
R⁶, R⁷, R^{6'} und R^{7'}, gleich oder verschieden, Wasserstoff, C₁-C₄-Alkyl, C₁-C₄-Alkyloxy, Hydroxy, -CF₃, -CHF₂, CN, NO₂ oder Halogen;
R^{x} und R^{x'} gleich oder verschieden Wasserstoff, C₁-C₄-Alkyl, C₁-C₄-Alkyloxy, Hydroxy, -CF₃, -CHF₂, CN, NO₂ oder Halogen
oder
R^{x} und R^{x'} gemeinsam eine Einfachbindung oder eine verbrückende Gruppe ausgewählt aus den Brücken -O-, -S-, -NH-, -CH₂-, -CH₂-CH₂-, -N(C₁-C₄-Alkyl)-, -CH(C₁-C₄-Alkyl)- und -C(C₁-C₄-Alkyl)₂-,
bedeuten, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

2. Verbindungen der allgemeinen Formel **1** nach Anspruch 1,
worin
n 1 oder 2;
A O, N oder eine Einfachbindung;
B ein zweibindiger Rest ausgewählt aus der Gruppe bestehend aus -CH₂-CH₂-, -CH=CH- und -CH₂-O-;
L ein zweibindiger Rest ausgewählt aus der Gruppe bestehend aus -CO-(CH₂)ₘ, -CO-(CH₂)ₘ-NH-CO-(CH₂)ₒ-, -CO-(CH₂)ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ;
m, o unabhängig voneinander 1 bis 10;
p, r unabhängig voneinander 2 bis 10;
R¹, R^{2,} R³ gleich oder verschieden, Wasserstoff, C₁-C₄-Alkyl, Halogen-C₁-C₄-alkylen, OH, -CF₃, CHF₂, HO-C₁₋₄-alkylen, -O-C₁-C₄-Alkyl, C₆-C₁₀-Aryl, C₆-C₁₀-Aryl-C₁-C₂-alkylen, C₆-C₁₀-Aryl-C₁-C₄-alkylen-O-, -COOH, -COOC₁-C₄-alkyl, -O-C₁-C₄-alkylen-COOH, -O-C₁-C₄-alkylen-COOC₁-C₄-alkyl, -NHSO₃H, -NHSO₂-C₁-C₄-alkyl, CN, NH₂, -NH-C₁-C₄-Alkyl, -N(C₁-C₄-Alkyl)₂, NO₂, -S-C₁-C₄-Alkyl, -SO₂-C₁-C₄-Alkyl, -SO-C₁-C₄-Alkyl, -O(CO)C₁-C₄-Alkyl, -COC₁-C₄-Alkyl, -NHCOC₁-C₄-Alkyl oder Halogen, besonders Fluor, Chlor oder Brom;
D ein zweibindiger Rest ausgewählt aus der Gruppe bestehend aus -CH₂-CH₂-, -CH=CH-, 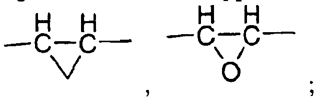
R⁴ Wasserstoff, C₁-C₄-Alkyl, C₆-C₁₀-Aryl, C₆-C₁₀-Aryl-C₁-C₂-alkylen oder die Gruppe 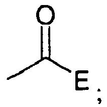
E ein Rest ausgewählt aus der Gruppe bestehend aus 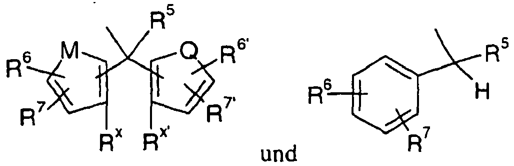
R⁵ Wasserstoff, Hydroxy, -C₁-C₄-Alkyl, -C₁-C₄-Alkyloxy, -CF₃, -CHF₂, HO-CH₂-, HO-CH₂-CH₂-, Fluor, Chlor oder Brom;
M und Q gleich oder verschieden, bevorzugt gleich, -O-, -S-, -NH- oder -CH=CH-;
R⁶, R⁷, R^{6'} und R^{7'}, gleich oder verschieden, Wasserstoff, C₁-C₄-Alkyl, C₁-C₄-Alkyloxy, Hydroxy, -CF₃, -CHF₂, CN, NO₂, Fluor, Chlor oder Brom;
R^{x} und R^{x'} gleich oder verschieden, Wasserstoff, C₁-C₄-Alkyl, C₁-C₄-Alkyloxy, Hydroxy, -CF₃, -CHF₂, CN, NO₂, Fluor, Chlor oder Brom
oder
R^{X} und R^{X'} gemeinsam eine Einfachbindung oder eine verbrückende Gruppe ausgewählt aus den Brücken -O-, -S-, -NH- und -CH₂-,
bedeuten, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

3. Verbindungen der allgemeinen Formel **1** nach Anspruch 1 oder 2,
worin
n 1 oder 2, bevorzugt 1;
A O, N oder eine Einfachbindung;
B ein zweibindiger Rest ausgewählt aus der Gruppe bestehend aus -CH₂-CH₂-, -CH=CH- und -CH₂-O-;
L ein zweibindiger Rest ausgewählt aus der Gruppe bestehend aus -CO-(CH₂)ₘ-, -CO-(CH₂)ₘ-NH-CO-(CH₂)ₒ-, -CO-(CH₂)ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ-
m, o unabhängig voneinander 1 bis 8;
p, r unabhängig voneinander 2 bis 8;
R¹, R^{2,} R³ gleich oder verschieden, Wasserstoff, C₁-C₄-Alkyl, C₁-C₄-Alkyloxy, Hydroxy, -CF₃, -CHF₂, Phenyl, Benzyl, -COOH, -COOMethyl, -O-CH₂-COOH, -O-CH₂-COOMethyl, -O-CH₂-COOEthyl, -O-CH₂-CH₂-COOH, -O-CH₂-CH₂-COOMethyl, -O-CH₂-CH₂-COOEthyl, CN, NO₂, Fluor, Chlor oder Brom;
D ein zweibindiger Rest ausgewählt aus der Gruppe bestehend aus -CH₂-CH₂-, -CH=CH-, 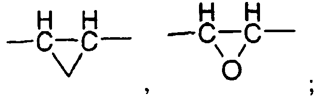
R⁴ Wasserstoff, Methyl, Ethyl, Phenyl, Benzyl oder die Gruppe 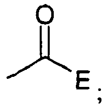
E ein Rest ausgewählt aus der Gruppe bestehend aus 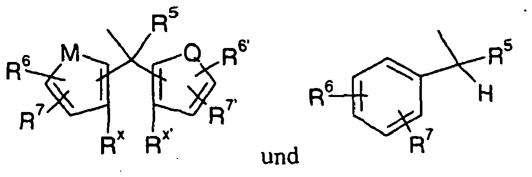
R⁵ Wasserstoff, Hydroxy, Methyl, Ethyl, Methyloxy, Ethyloxy, -CF₃, HO-CH₂-, HO-CH₂-CH₂- oder Fluor;
M und Q gleich oder verschieden, bevorzugt gleich, -S- oder -CH=CH-;
R⁶, R⁷, R^{6'} und R^{7'}, gleich oder verschieden, Wasserstoff, Methyl, Methyloxy, -CF₃ oder Fluor;
R^{x} und R^{x'} gleich oder verschieden Wasserstoff, Methyl, Methyloxy, -CF₃ oder Fluor
oder
R^{x} und R^{x'} gemeinsam eine Einfachbindung oder die verbrückende Gruppe -O-,
bedeuten, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

4. Verbindungen der allgemeinen Formel **1** nach Anspruch 1, 2 oder 3,
worin n 1, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

5. Verbindungen der allgemeinen Formel **1** nach einem der Ansprüche 1 bis 4, worin A für O (Sauerstoff) oder N (Stickstoff) steht, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

6. Verbindungen der allgemeinen Formel **1** nach einem der Ansprüche 1 bis 5, worin B für -CH=CH- oder -CH₂-O- steht, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

7. Verbindungen der allgemeinen Formel **1** nach einem der Ansprüche 1 bis 6, worin
L ein zweibindiger Rest ausgewählt aus der Gruppe bestehend aus -CO-(CH₂)₁₋₆-, -CO-(CH₂)₁₋₆-NH-CO-(CH₂)₁₋₄-, -CO-(CH₂)₁₋₆-CO-NH-(CH₂)₁₋₄-, -CO-(CH₂)₁₋₆-O-(CH₂)₂₋₆-, -(CH₂)₂₋₆-, -(CH₂)₂₋₆-O-(CH₂)₂₋₆-, -(CH₂)₂₋₆-NH-CO-(CH₂)₁₋₄- bedeutet,
gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

8. Verbindungen der Formel **1** nach einem der Ansprüche 1 bis 7, **dadurch gekennzeichnet, dass** sie in Form eines ihrer Säureadditionssalze mit pharmakologisch unbedenklichen Säuren vorliegen, welches ausgewählt ist aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat.

9. Verbindungen der Formel **1** nach einem der Ansprüche 1 bis 8, **dadurch gekennzeichnet, dass** sie in Form der R-Enantiomere der allgemeinen Formel ***R*-1** 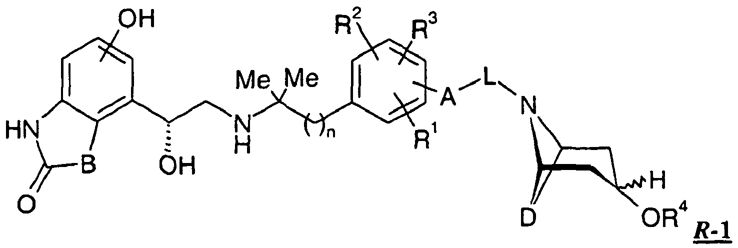 vorliegen, gegebenenfalls in Form ihrer Säureadditionssalze mit pharmakologisch unbedenklichen Säuren sowie gegebenenfalls in Form der Solvate und/oder Hydrate.

10. Verbindungen der Formel **1** nach einem der Ansprüche 1 bis 8, **dadurch gekennzeichnet, dass** sie in Formn der Regioisomere der allgemeinen Formel ***Regio*-1a** 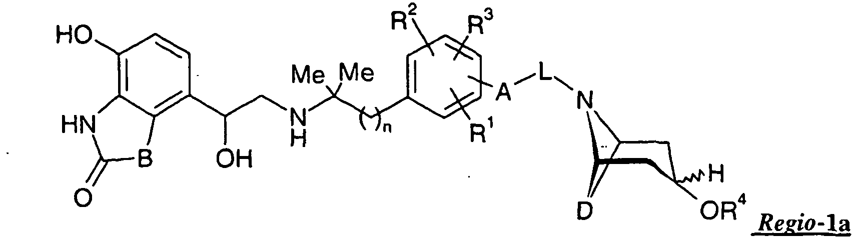 vorliegen, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

11. Verbindungen der Formel **1** nach einem der Ansprüche 1 bis 8, **dadurch gekennzeichnet, dass** sie in Form der Regioisomere der allgemeinen Formel ***Regio-1b*** 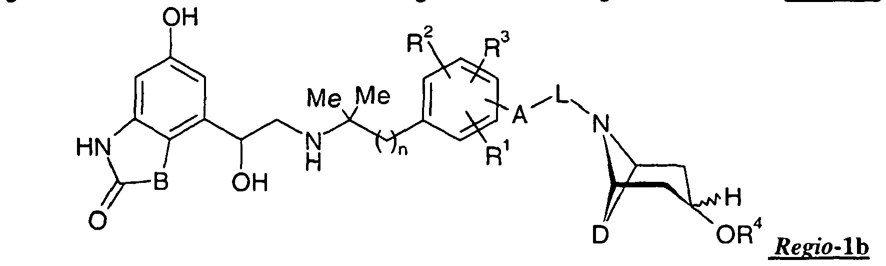 vorliegen, gegebenenfalls in Form der einzelnen Enantiomere oder gegebenenfalls Diastereomere, Mischungen der einzelnen Enantiomere oder gegebenenfalls Diastereomere oder Racemate, gegebenenfalls in Form ihrer Säureadditionssalze mit pharmakologisch unbedenklichen Säuren sowie gegebenenfalls in Form der Solvate und/oder Hydrate.

12. Verbindungen der allgemeinen Formel **1** gemäß einem der Ansprüche 1 bis 11 als Arzneimittel.

13. Verwendung der Verbindungen der allgemeinen Formel **1** gemäß einem der Ansprüche 1 bis 12 zur Herstellung eines Arzneimittels zur Behandlung von Atemwegserkrankungen.

14. Pharmazeutischen Formulierung, **gekennzeichnet durch** einen Gehalt einer Verbindung der Formel **1** gemäß einem der Ansprüche 1 bis 12.

15. Zwischenprodukte der allgemeinen Formel **4** 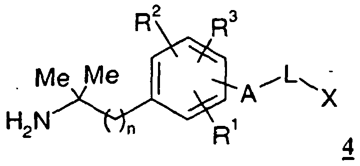 worin
X Chlor, Brom, Iod, Methansulfonat, Trifluormethansulfonat, p-Toluolsulfonat, oder eine durch eine geeignete Schutzgruppe geschützte Aminofunktion, vorzugsweise -NH-Boc, -NH-Cbz, -NH-Fmoc, -NH-Teoc, oder -NH-Alloc
bedeuten und worin n, A, L, R¹, R² und R³ die in den Ansprüchen 1 bis 8 genannten Bedeutungen aufweisen können.

16. Zwischenprodukte der allgemeinen Formel **5** 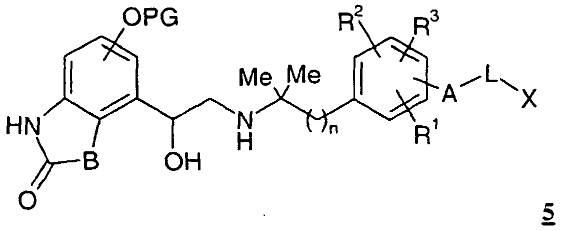 worin
OPG eine durch eine Schutzgruppe PG geschützte Hydroxyfunktion, vorzugsweise -O-C₁-C₄-Alkyl, -O-Benzyl oder -O-CH₂-O-C₁-C₄-Alkyl, bevorzugt -O-Methyl, -O-Ethyl, -O-Benzyl oder -O-CH₂-O-Methyl, besonders bevorzugt -O-Ethyl oder -O-Benzyl, besonders bevorzugt -O-Benzyl
bedeutet und worin n, A, L, X, R¹, R² und R³ die in den Ansprüchen 1 bis 8 und 15 genannten Bedeutungen aufweisen können.

17. Zwischenprodukte der allgemeinen Formel **7** 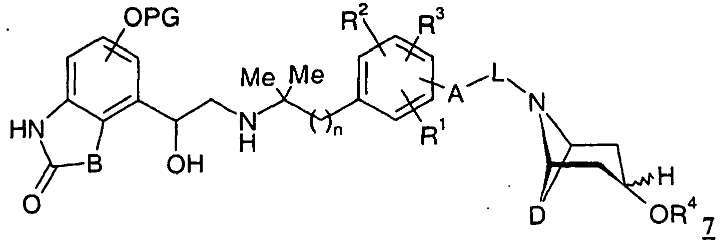 worin n, A, L, D, X, OPG, R¹, R² und R³ die in den Ansprüchen 1 bis 8, 15 und 16 genannten Bedeutungen aufweisen können.

## Claims

1. Compounds of general formula **1** 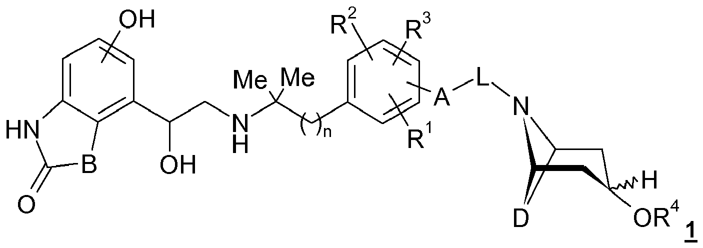 wherein
n denotes 1, 2 or 3, preferably 1 and 2;
A denotes O, N or a single bond;
B denotes a double-bonded group selected from among -CH₂-CH₂-, -CH=CH- and -CH₂-O-;
L denotes a double-bonded group selected from among -CO-(CH₂)ₘ-, -CO-(CH₂)ₘ-NH-CO-(CH₂)ₒ-, -CO-(CH₂)ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ-, -(CH₂)ₚ-CO-NH-(CH₂)ₒ-, SO₂-(CH₂)ₘ-, -SO₂-(CH₂)ₘ-NH-CO-(CH₂)ₒ-, -SO₂-(CH₂)ₘ-CO-NH-(CH₂)ₒ-, -SO₂-(CH₂)ₘ-O-(CH₂)ₚ-;
m, o independently of one another denote 1 to 12;
p, r independently of one another denote 2 to 12;
R¹, R^{2,} R³ which may be identical or different, denote hydrogen, C₁-C₆-alkyl, halogen-C₁-C₆-alkylene, OH, -CF₃, CHF₂, HO-C₁₋₆-alkylene, -O-C₁-C₆-alkyl, C₆-C₁₀-aryl, C₆-C₁₀-aryl-C₁-C₄-alkylene, C₆-C₁₀-aryl-C₁-C₆-alkylene-O, -COOH, -COOC₁-C₆-alkyl, -O-C₁-C₆-alkylene-COOH, -O-C₁-C₆-alkylene-COOC₁-C₆-alkyl, -NHSO₃H, -NHSO₂-C₁-C₆-alkyl, CN, NH₂, -NH-C₁-C₆-alkyl, -N(C₁-C₆-alkyl)₂, NO₂, -S-C₁-C₆-alkyl, -SO₂-C₁-C₆-alkyl, -SO-C₁-C₆-alkyl, -O(CO)C₁-C₆-alkyl, -COC₁-C₆-alkyl, -NHCOC₁-C₆-alkyl or halogen, particularly fluorine, chlorine or bromine;
D denotes a double-bonded group selected from among -CH₂-CH₂-, -CH=CH-, 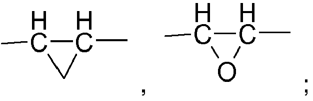
R⁴ denotes hydrogen, C₁-C₆-alkyl, C₆-C₁₀-aryl, C₆-C₁₀-aryl-C₁-C₄-alkylene or the group 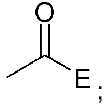
E denotes a group selected from among 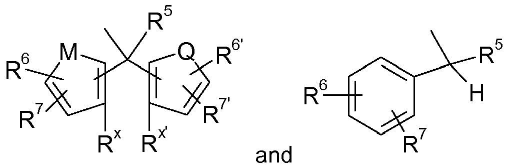
R⁵ denotes hydrogen, hydroxy, -C₁-C₄-alkyl, - C₁-C₄-alkyloxy, - C₁-C₄-alkylene-halogen, -O-C₁-C₄-alkylene-halogen, - C₁-C₄-alkylene-OH, -CF₃, CHF₂, -C₁-C₄-alkylene- C₁-C₄-alkyloxy, -O-CO C₁-C₄-alkyl, -O-CO C₁-C₄-alkylene-halogen, -C₁-C₄-alkylene-C₃-C₆-cycloalkyl, -O-COCF₃ or halogen;
M and Q which may be identical or different, preferably identical, denote -O-, -S-, -NH-, -CH₂-, -CH=CH- or -N(C₁-C₄-alkyl)-;
R⁶, R⁷, R^{6'} and R^{7'}, which may be identical or different, denote hydrogen, C₁-C₄-alkyl, C₁-C₄-alkyloxy, hydroxy, -CF₃, -CHF₂, CN, NO₂ or halogen;
R^{x} and R^{x'} which may be identical or different denote hydrogen, C₁-C₄-alkyl, C₁-C₄-alkyloxy, hydroxy, -CF₃, -CHF₂, CN, NO₂ or halogen
or
R^{x} and R^{x'} together denote a single bond or a bridging group selected from the bridges -O-, -S-, -NH-, -CH₂-, -CH₂-CH₂-, -N(C₁-C₄-alkyl)-, -CH(C₁-C₄-alkyl)- and -C(C₁-C₄-alkyl)₂-,
optionally in the form of the individual enantiomers or optionally diastereomers, mixtures of the individual enantiomers or optionally diastereomers or racemates, optionally in the form of the acid addition salts thereof with pharmacologically acceptable acids as well as optionally in the form of the solvates and/or hydrates thereof.

2. Compounds of general formula **1** according to claim 1,
wherein
n denotes 1 or 2;
A denotes O, N or a single bond;
B denotes a double-bonded group selected from among -CH₂-CH₂-, -CH=CH- and -CH₂-O-;
L denotes a double-bonded group selected from among -CO-(CH₂)ₘ-, -CO-(CH₂)ₘ-NH-CO-(CH₂)ₒ-, -CO-(CH₂)ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-,-(CH₂)ₚ-NH-CO-(CH₂)ₒ-;
m, o independently of one another denote 1 to 10;
p, r independently of one another denote 2 to 10;
R¹, R^{2,} R³ which may be identical or different, denote hydrogen, C₁-C₄-alkyl, halogen-C₁-C₄-alkylene, OH, -CF₃, CHF₂, HO-C₁₋₄-alkylene, -O-C₁-C₄-alkyl, C₆-C₁₀-aryl, C₆-C₁₀-aryl-C₁-C₂-alkylene, C₆-C₁₀-aryl-C₁-C₄-alkylene-O-, -COOH, -COOC₁-C₄-alkyl, -O-C₁-C₄-alkylene-COOH, -O-C₁-C₄-alkylene-COOC₁-C₄-alkyl, -NHSO₃H, -NHSO₂-C₁-C₄-alkyl, CN, NH₂, -NH-C₁-C₄-alkyl, -N(C₁-C₄-alkyl)₂, NO₂, -S-C₁-C₄-alkyl, -SO₂-C₁-C₄-alkyl, -SO-C₁-C₄-alkyl, -O(CO)C₁-C₄-alkyl, -COC₁-C₄-alkyl, -NHCOC₁-C₄-alkyl or halogen, particularly fluorine, chlorine or bromine;
D denotes a double-bonded group selected from among -CH₂-CH₂-, -CH=CH-, 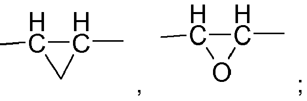
R⁴ denotes hydrogen, C₁-C₄-alkyl, C₆-C₁₀-aryl, C₆-C₁₀-aryl-C₁-C₂-alkylene or the group 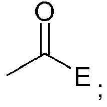
E denotes a group selected from among 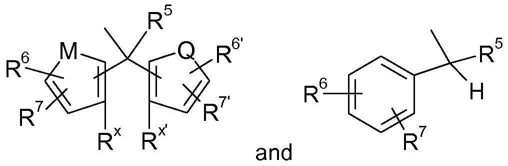
R⁵ denotes hydrogen, hydroxy, -C₁-C₄-alkyl, -C₁-C₄-alkyloxy, -CF₃, -CHF₂, HO-CH₂-, HO-CH₂-CH₂-, fluorine, chlorine or bromine;
M and Q which may be identical or different, preferably identical, denote -O-, -S-, -NH-or -CH=CH-;
R⁶, R⁷, R^{6'} and R^{7'}, which may be identical or different, denote hydrogen, C₁-C₄-alkyl, C₁-C₄-alkyloxy, hydroxy, -CF₃, -CHF₂, CN, NO₂, fluorine, chlorine or bromine;
R^{x} and R^{x'} which may be identical or different, denote hydrogen, C₁-C₄-alkyl, C₁-C₄-alkyloxy, hydroxy, -CF₃, -CHF₂, CN, NO₂, fluorine, chlorine or bromine
or
R^{X} and R^{X'} together denote a single bond or a bridging group selected from the bridges -O-, -S-, -NH- and -CH₂- ,
optionally in the form of the individual enantiomers or optionally diastereomers, mixtures of the individual enantiomers or optionally diastereomers or racemates, optionally in the form of the acid addition salts thereof with pharmacologically acceptable acids as well as optionally in the form of the solvates and/or hydrates thereof.

3. Compounds of general formula **1** according to claim 1 or 2,
wherein
n denotes 1 or 2, preferably 1;
A denotes O, N or a single bond;
B denotes a double-bonded group selected from among -CH₂-CH₂-, -CH=CH- and -CH₂-O-;
L denotes a double-bonded group selected from among -CO-(CH₂)ₘ-, -CO-(CH₂)ₘ-NH-CO-(CH₂)ₒ-, -CO-(CH₂)ₘ-CO-NH-(CH₂)ₒ-, -CO-(CH₂)ₘ-O-(CH₂)ₚ, -(CH₂)ₚ-, -(CH₂)ₚ-O-(CH₂)ᵣ-, -(CH₂)ₚ-NH-CO-(CH₂)ₒ-
m, o independently of one another denote 1 to 8;
p, r independently of one another denote 2 to 8;
R¹, R^{2,} R³ which may be identical or different, denote hydrogen, C₁-C₄-alkyl, C₁-C₄-alkyloxy, hydroxy, -CF₃, -CHF₂, phenyl, benzyl, -COOH, -COOmethyl, -O-CH₂-COOH, -O-CH₂-COOmethyl, -O-CH₂-COOethyl, -O-CH₂-CH₂-COOH, -O-CH₂-CH₂-COOmethyl, -O-CH₂-CH₂-COOethyl, CN, NO₂, fluorine, chlorine or bromine;
D denotes a double-bonded group selected from among -CH₂-CH₂-, -CH=CH-, 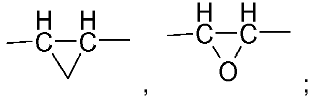
R⁴ denotes hydrogen, methyl, ethyl, phenyl, benzyl or the group 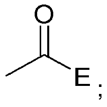
E denotes a group selected from among 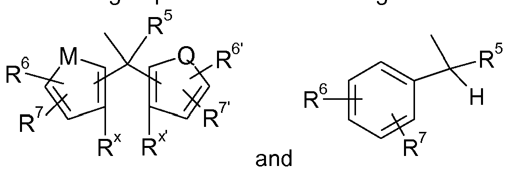
R⁵ denotes hydrogen, hydroxy, methyl, ethyl, methyloxy, ethyloxy, -CF₃, HO-CH₂-, HO-CH₂-CH₂- or fluorine;
M and Q which may be identical or different, preferably identical, denote -S- or -CH=CH-;
R⁶, R⁷, R^{6'} and R^{7'}, which may be identical or different, denote hydrogen, methyl, methyloxy, -CF₃ or fluorine;
R^{x} and R^{x'} which may be identical or different denote hydrogen, methyl, methyloxy, -CF₃ or fluorine
or
R^{x} and R^{x'} together denote a single bond or the bridging group -O-,
optionally in the form of the individual enantiomers or optionally diastereomers, mixtures of the individual enantiomers or optionally diastereomers or racemates, optionally in the form of the acid addition salts thereof with pharmacologically acceptable acids as well as optionally in the form of the solvates and/or hydrates thereof.

4. Compounds of general formula **1** according to claim 1, 2 or 3,
wherein n denotes 1, optionally in the form of the individual enantiomers or optionally diastereomers, mixtures of the individual enantiomers or optionally diastereomers or racemates, optionally in the form of the acid addition salts thereof with pharmacologically acceptable acids as well as optionally in the form of the solvates and/or hydrates thereof.

5. Compounds of general formula **1** according to one of claims 1 to 4,
wherein A denotes O (oxygen) or N (nitrogen), optionally in the form of the individual enantiomers or optionally diastereomers, mixtures of the individual enantiomers or optionally diastereomers or racemates, optionally in the form of the acid addition salts thereof with pharmacologically acceptable acids as well as optionally in the form of the solvates and/or hydrates thereof.

6. Compounds of general formula **1** according to one of claims 1 to 5, wherein B denotes -CH=CH- or -CH₂-O-, optionally in the form of the individual enantiomers or optionally diastereomers, mixtures of the individual enantiomers or optionally diastereomers or racemates, optionally in the form of the acid addition salts thereof with pharmacologically acceptable acids as well as optionally in the form of the solvates and/or hydrates thereof.

7. Compounds of general formula **1** according to one of claims 1 to 6, wherein
L denotes a double-bonded group selected from among -CO-(CH₂)₁₋₆-, -CO-(CH₂)₁₋₆-NH-CO-(CH₂)₁₋₄-, -CO-(CH₂)₁₋₆-CO-NH-(CH₂)₁₋₄-, -CO-(CH₂)₁₋₆-O-(CH₂)₂₋₆-, -(CH₂)₂₋₆-, -(CH₂)₂₋₆-O-(CH₂)₂₋₆-, -(CH₂)₂₋₆-NH-CO-(CH₂)₁₋₄-,
optionally in the form of the individual enantiomers or optionally diastereomers, mixtures of the individual enantiomers or optionally diastereomers or racemates, optionally in the form of the acid addition salts thereof with pharmacologically acceptable acids as well as optionally in the form of the solvates and/or hydrates thereof.

8. Compounds of formula **1** according to one of claims 1 to 7, **characterised in that** they are in the form of one of the acid addition salts thereof with pharmacologically acceptable acids, which is selected from among the hydrochloride, hydrobromide, hydroiodide, hydrosulphate, hydrophosphate, hydromethanesulphonate, hydronitrate, hydromaleate, hydroacetate, hydrobenzoate, hydrocitrate, hydrofumarate, hydrotartrate, hydroxalate, hydrosuccinate, hydrobenzoate and hydro-p-toluenesulphonate.

9. Compounds of formula **1** according to one of claims 1 to 8, **characterised in that** they are in the form of the R-enantiomers of general formula ***R*-1** 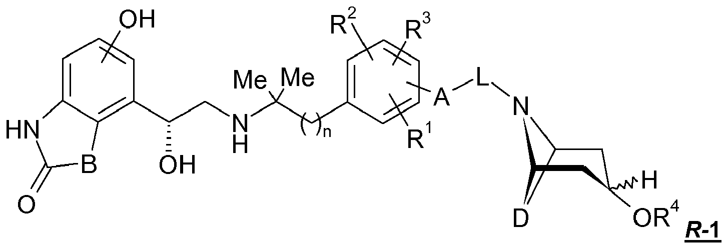 optionally in the form of the acid addition salts thereof with pharmacologically acceptable acids as well as optionally in the form of the solvates and/or hydrates thereof.

10. Compounds of formula **1** according to one of claims 1 to 8, **characterised in that** they are in the form of the regioisomers of general formula ***Regio*-1a** 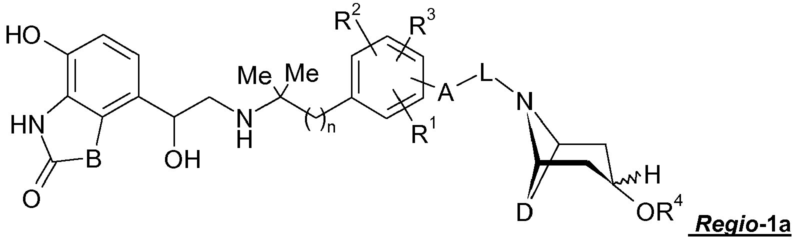 optionally in the form of the individual enantiomers or optionally diastereomers, mixtures of the individual enantiomers or optionally diastereomers or racemates, optionally in the form of the acid addition salts thereof with pharmacologically acceptable acids as well as optionally in the form of the solvates and/or hydrates thereof.

11. Compounds of formula **1** according to one of claims 1 to 8, **characterised in that** they are in the form of the regioisomers of general formula ***Regio*-1b** 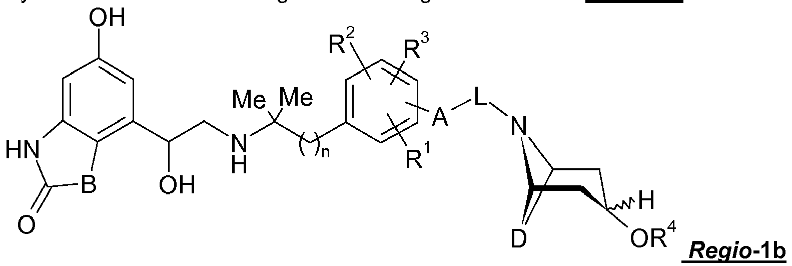 optionally in the form of the individual enantiomers or optionally diastereomers, mixtures of the individual enantiomers or optionally diastereomers or racemates, optionally in the form of the acid addition salts thereof with pharmacologically acceptable acids as well as optionally in the form of the solvates and/or hydrates thereof.

12. Compounds of general formula **1** according to one of claims 1 to 11 as medicaments.

13. Use of the compounds of general formula **1** according to one of claims 1 to 12 for preparing a medicament for the treatment of respiratory complaints.

14. Pharmaceutical formulation, **characterised in that** it contains a compound of formula **1** according to one of claims 1 to 12.

15. Intermediate products of general formula **4** 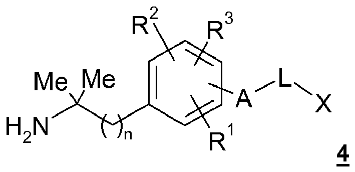 wherein
X denotes chlorine, bromine, iodine, methanesulphonate, trifluoromethanesulphonate, p-toluenesulphonate, or an amino function protected by a suitable protective group, preferably -NH-Boc, -NH-Cbz, -NH-Fmoc, -NH-Teoc or -NH-Alloc,
and wherein n, A, L, R¹, R² and R³ may have the meanings given in claims 1 to 8.

16. Intermediate products of general formula **5** 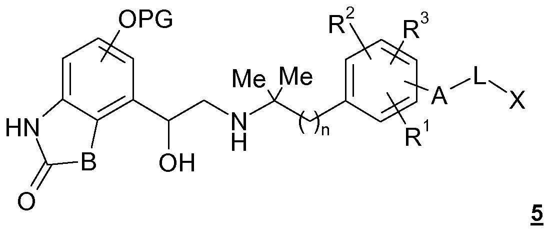 wherein
OPG denotes a hydroxy function protected by a protective group PG, preferably -O-C₁-C₄-alkyl, -O-benzyl or -O-CH₂-O-C₁-C₄-alkyl, preferably -O-methyl, -O-ethyl, -O-benzyl or -O-CH₂-O-methyl, particularly preferably -O-ethyl or -O-benzyl, particularly preferably -O-benzyl
and wherein n, A, L, X, R¹, R² and R³ may have the meanings given in claims 1 to 8 and 15.

17. Intermediate products of general formula 7 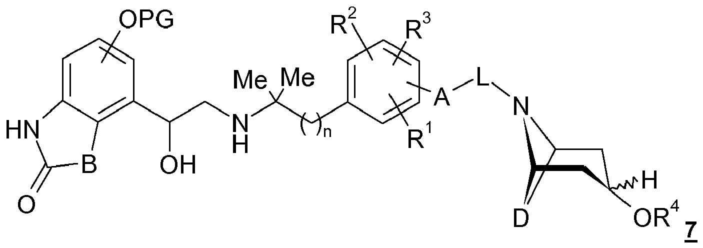 wherein n, A, L, D, X, OPG, R¹, R² and R³ may have the meanings given in claims 1 to 8, 15 and 16.

## Revendications

1. Composés de formule générale I 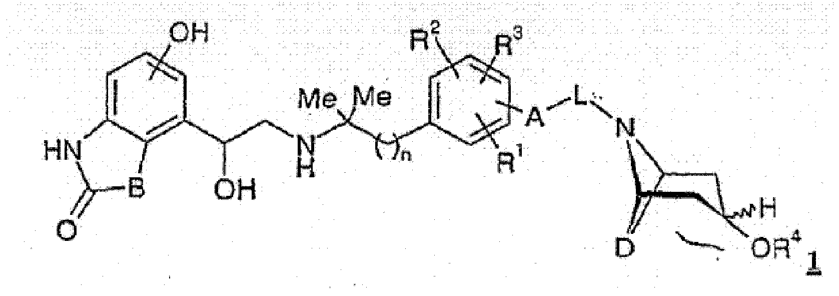 dans laquelle
n est 1, 2 ou 3, de préférence 1 et 2 ;
A est 0, N ou une liaison simple ;
B est un radical à double liaison choisi dans le groupe comprenant -CH₂-CH₂-, -CH=CH- et -CH₂-O- ;
L est un radical à double liaison choisi dans le groupe comprenant -CO-(CH₂)ₘ-, -CO-(CH₂)ₘ-NH-CO-(CH₂)ₒ-, -CO-(CH₂) ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ-, -(CH₂)ₚ-CO-NH-(CH₂)ₒ-, SO₂-(CH₂)ₘ-, -SO₂-(CH₂) ₘ-NH-CO-(CH₂)ₒ-, -SO₂-(CH₂)ₘ-CO-NH-(CH₂)ₒ-, SO₂-(CH₂)ₘ-O-(CH₂)ₚ- ;
m, o sont indépendamment l'un de l'autre 1 à 12 ;
p, r sont indépendamment l'un de l'autre 2 à 12 ;
R¹, R², R³ identiques ou différents sont un atome d'hydrogène, un groupe alkyle en C₁ à C₆, halogéno-alkylène en C₁ à C₆, OH, -CF₃, CHF₂, HO-alkylène en C₁ à C₆, -0-alkyle en C₁ à C₆, aryle en C₆ à C₁₀, aryle en C₆ à C₁₀-alkylène en C₁ à C₄, aryle en C₆ à C₁₀-alkylène en C₁ à C₆-O-, -COOH,-COO-alkyle en C₁ à C₆, -0-alkylène en C₁ à C₆-COOH, -0-alkylène en C₁ à C₆-COO-alkyle en C₁ à C₆,-NHSO₃H, -NHSO₂-alkyle en C₁ à C₆, CN, NH₂, -NH-alkyle en C₁ à C₆, -N(alkyle en C₁ à C₆) ₂, NO₂, -S-alkyle en C₁ à C₆, -SO₂-alkyle en C₁ à C₆, -SO-alkyle en C₁ à C₆, -O(CO)alkyle en C₁ à C₆, -CO-alkyle en C₁ à C₆, -NHCO-alkyle en C₁ à C₆ ou un atome d'halogène, en particulier, de fluor, de chlore ou de brome ;
D est un radical à double liaison choisi dans le groupe comprenant -CH₂-CH₂-, -CH=CH-, 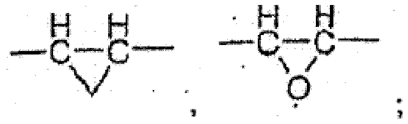
R⁴ est un atome d'hydrogène, un groupe alkyle en C₁ à C₆, aryle en C₆ à C₁₀, aryle en C₆ à C₁₀-alkylène en C₁ à C₄ ou le groupe 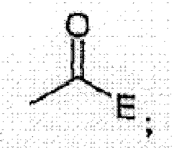
E est un radical choisi dans le groupe comprenant 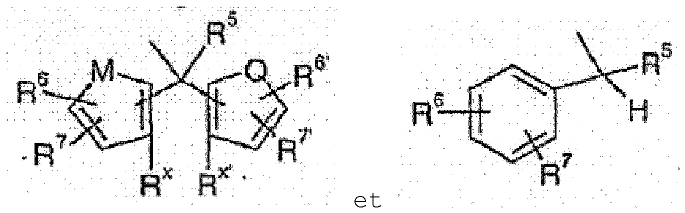
R⁵ est un atome d'hydrogène, un groupe hydroxy, alkyle en C₁ à C₄, alkyloxy en C₁ à C₄, alkylène en C₁ à C₄-halogéno, -0-alkylène en C₁ à C₄-halogéno, alkylène en C₁ à C₄-OH, -CF₃, CHF₂, alkylène en C₁ à C₄-alkyloxy en C₁ à C₄, -0-CO-alkyle en C₁ à C₄, -0-CO-alkylène en C₁ à C₄-halogéno, alkylène en C₁ à C₄-cycloalkyle en C₃ à C₆, -O-COCF₃ ou un atome d'halogène ;
M et Q, identiques ou différents, de préférence identiques, sont -O-, -S-, -NH-, -CH₂-, -CH=CH- ou -N(alkyle en C₁ à C₄) - ;
R⁶, R⁷, R^{6'} et R^{7'}, identiques ou différents, sont un atome d'hydrogène, un groupe alkyle en C₁ à C₄, alkyloxy en C₁ à C₄, hydroxy, -CF₃, -CHF₂, CN, NO₂ ou un atome d'halogène ;
R^{x} et R^{x'}, identiques ou différents, sont un atome d'hydrogène, un groupe alkyle en C₁ à C₄, alkyloxy en C₁ à C₄, hydroxy, -CF₃, -CHF₂, CN, NO₂ ou un atome d'halogène, ou
R^{x} et R^{x'} sont conjointement une liaison simple ou un groupe ponté choisi parmi les ponts -O-, -S-,-NH-, -CH₂-, -CH₂-CH₂-, -N(alkyle en C₁ à C₄)-,-CH(alkyle en C₁ à C₄)- et -C(alkyle en C₁ à C₄)₂-,
éventuellement sous la forme des énantiomères individuels ou éventuellement des diastéréomères, de mélanges des énantiomères individuels ou éventuellement des diastéréomères ou des racémates, éventuellement sous la forme de leurs sels d'addition acides avec des acides pharmacologiquement acceptables et éventuellement sous la forme de solvates et/ou d'hydrates.

2. Composés de formule générale 1 selon la revendication 1,
dans laquelle
n est 1 ou 2 ;
A est 0, N ou une liaison simple ;
B est un radical à double liaison choisi dans le groupe comprenant -CH₂-CH₂-, -CH=CH- et -CH₂-O- ;
L est un radical à double liaison choisi dans le groupe comprenant -CO- (CH₂) ₘ-, -CO- (CH₂) ₘ-NH-CO-(CH₂)ₒ-, -CO-(CH₂)ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ- ;
m, o sont indépendamment l'un de l'autre 1 à 10 ;
p, r sont indépendamment l'un de l'autre 2 à 10 ;
R¹, R², R³, identiques ou différents sont un atome d'hydrogène, un groupe alkyle en C₁ à C₄, halogéno-alkylène en C₁ à C₄, OH, -CF₃, CHF₂, HO-alkylène en C₁ à C₄, -0-alkyle en C₁ à C₄, aryle en C₆ à C₁₀, aryle en C₆ à C₁₀-alkylène en C₁ à C₂, aryle en C₆ à C₁₀-alkylène en C₁ à C₄-O-, -COOH,-COO-alkyle en C₁ à C₄, -0-alkylène en C₁ à C₄-COOH, -0-alkylène en C₁ à C₄-COO-alkyle en C₁ à C₄,-NHSO₃H, -NHSO₂-alkyle en C₁ à C₄, CN, NH₂, -NH-alkyle en C₁ à C₄, -N (alkyle en C₁ à C₄) ₂, NO₂, -S-alkyle en C₁ à C₄, -SO₂-alkyle en C₁ à C₄, -SO-alkyle en C₁ à C₄, -O(CO)alkyle en C₁ à C₄, -CO-alkyle en C₁ à C₄, -NHCO-alkyle en C₁ à C₄ ou un atome d'halogène, en particulier, de fluor, de chlore ou de brome ;
D est un radical à double liaison choisi dans le groupe comprenant -CH₂-CH₂-, -CH=CH-, 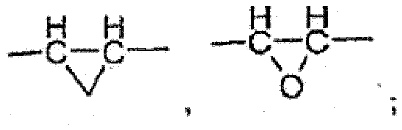
R⁴ est un atome d'hydrogène, un groupe alkyle en C₁ à C₄, aryle en C₆ à C₁₀, aryle en C₆ à C₁₀-alkylène en C₁ à C₂ ou le groupe 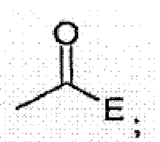
E est un radical choisi dans le groupe comprenant 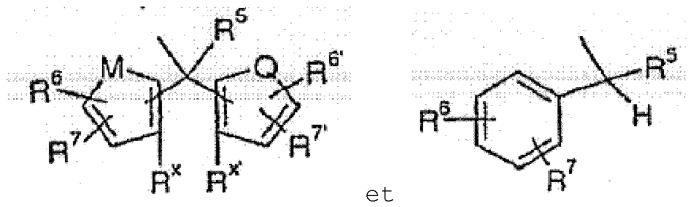
R⁵ est un atome d'hydrogène, un groupe hydroxy, alkyle en C₁ à C₄, alkyloxy en C₁ à C₄, -CF₃, CHF₂, HO-CH₂-, -HO-CH₂-CH₂-, un atome de fluor, de chlore ou de brome ;
M et Q, identiques ou différents, de préférence identiques, sont -O-, -S-, -NH- ou -CH=CH- ;
R⁶, R⁷, R^{6'} et R^{7'}, identiques ou différents, sont un atome d'hydrogène, un groupe alkyle en C₁ à C₄, alkyloxy en C₁ à C₄, hydroxy, -CF₃, -CHF₂, CN, NO₂, un atome de fluor, de chlore ou de brome ;
R^{x} et R^{x'}, identiques ou différents, sont un atome d'hydrogène, un groupe alkyle en C₁ à C₄, alkyloxy en C₁ à C₄, hydroxy, -CF₃, -CHF₂, CN, NO₂, un atome de fluor, de chlore ou de brome,
ou
R^{x} et R^{x'} sont conjointement une liaison simple ou un groupe ponté choisi parmi les ponts -O-, -S-,-NH- et -CH₂-,
éventuellement sous la forme des énantiomères individuels ou éventuellement, des diastéréomères, de mélanges des énantiomères individuels ou éventuellement des diastéréomères ou des racémates, éventuellement sous la forme de leurs sels d'addition acides avec des acides pharmacologiquement acceptables et éventuellement sous la forme de solvates et/ou d'hydrates.

3. Composés de formule générale 1 selon la revendication 1 ou 2,
dans laquelle
n est 1 ou 2, de préférence, 1 ;
A est 0, N ou une liaison simple ;
B est un radical à double liaison choisi dans le groupe comprenant -CH₂-CH₂-, -CH=CH- et -CH₂-O- ;
L est un radical à double liaison choisi dans le groupe comprenant -CO- (CH₂) ₘ-, -CO- (CH₂) ₘ-NH-CO-(CH₂)ₒ-, -CO-(CH₂) ₘ-CO-NH-(CH₂)ₒ-, -CO-(CH₂)ₘ-O-(CH₂)ₚ-, -(CH₂)ₚ-, -(CH₂)ₚ-O-(CH₂)ᵣ-, -(CH₂)ₚ-NH-CO-(CH₂)ₒ- ;
m, o sont indépendamment l'un de l'autre 1 à 8 ;
p, r sont indépendamment l'un de l'autre 2 à 8 ;
R¹, R², R³ identiques ou différents sont un atome d'hydrogène, un groupe alkyle en C₁ à C₄, alkyloxy en C₁ à C₄, hydroxy, -CF₃, -CHF₂, phényle, benzyle, -COOH, -COO-méthyle, -O-CH₂-COOH, -O-CH₂-COO-méthyle, -O-CH₂-COO-éthyle, -O-CH₂-CH₂-COOH, -O-CH₂-CH₂-COO-méthyle, -O-CH₂-CH₂-COO-éthyle, CN, NO₂, un atome de fluor, de chlore ou de brome ;
D est un radical à double liaison choisi dans le groupe comprenant -CH₂-CH₂-, -CH=CH-, 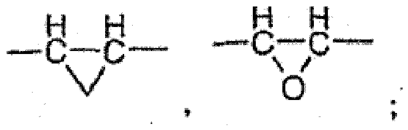
R⁴ est un atome d'hydrogène, un groupe méthyle, éthyle, phényle, benzyle ou le groupe 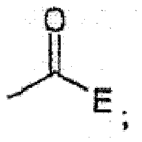
E est un radical choisi dans le groupe comprenant 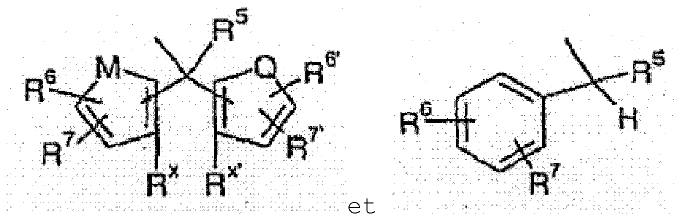
R⁵ est un atome d'hydrogène, un groupe hydroxy, méthyle, éthyle, méthyloxy, éthyloxy, -CF₃, HO-CH₂, HO-CH₂-CH₂- ou un atome de fluor ;
M et Q, identiques ou différents, de préférence, identiques, sont -S- ou -CH=CH- ;
R⁶, R⁷, R^{6'} et R^{7'}, identiques ou différents, sont un atome d'hydrogène, un groupe méthyle, méthyloxy,-CF₃ ou un atome de fluor ;
R^{x} et R^{x'}, identiques ou différents, sont un atome d'hydrogène, un groupe méthyle, méthyloxy, -CF₃ ou un atome de fluor,
ou
R^{x} et R^{x'} sont conjointement une liaison simple ou
le groupe ponté -O-, éventuellement sous la forme des énantiomères individuels ou éventuellement, des diastéréomères, de mélanges des énantiomères individuels ou éventuellement des diastéréomères ou des racémates, éventuellement sous la forme de leurs sels d'addition acides avec des acides pharmacologiquement acceptables et éventuellement sous la forme de solvates et/ou d'hydrates.

4. Composés de formule générale 1 selon la revendication 1, 2 ou 3, dans lesquels n est 1, éventuellement sous la forme des énantiomères individuels ou éventuellement, des diastéréomères, de mélanges des énantiomères individuels ou éventuellement des diastéréomères ou des racémates, éventuellement sous la forme de leurs sels d'addition acides avec des acides pharmacologiquement acceptables et éventuellement sous la forme de solvates et/ou d'hydrates.

5. Composés de formule générale 1 selon l'une des revendications 1 à 4, dans lesquels A désigne 0 (l'oxygène) ou N (l'azote), éventuellement sous la forme des énantiomères individuels ou éventuellement, des diastéréomères, de mélanges des énantiomères individuels ou éventuellement des diastéréomères ou des racémates, éventuellement sous la forme de leurs sels d'addition acides avec des acides pharmacologiquement acceptables et éventuellement sous la forme de solvates et/ou d'hydrates.

6. Composés de formule générale 1 selon l'une des revendications 1 à 5, dans lesquels B désigne -CH=CHou -CH₂-O-, éventuellement sous la forme des énantiomères individuels ou éventuellement, des diastéréomères, de mélanges des énantiomères individuels ou éventuellement des diastéréomères ou des racémates, éventuellement sous la forme de leurs sels d'addition acides avec des acides pharmacologiquement acceptables et éventuellement sous la forme de solvates et/ou d'hydrates.

7. Composés de formule générale 1 selon l'une des revendications 1 à 6,
dans laquelle
L est un radical à double liaison choisi dans le groupe comprenant -CO-(CH₂)_{1 à 6}-, -CO-(CH₂)_{1 à 6}-NH-CO-(CH₂)_{1 à 4}-, -CO-(CH₂)_{1 à 6}-CO-NH-(CH₂)_{1 à 4}-, -CO-(CH₂)_{1 à 6} -O-(CH₂)₂ à ₆-, -(CH₂)_{2 à 6}-, -(CH₂)_{2 à 6} -O-(CH₂)_{2 à 6}-, -(CH₂)_{2 à 6} -NH-CO-(CH₂)_{1 à 4}-,
éventuellement sous la forme des énantiomères individuels ou éventuellement, des diastéréomères, de mélanges des énantiomères individuels ou éventuellement des diastéréomères ou des racémates, éventuellement sous la forme de leurs sels d'addition acides avec des acides pharmacologiquement acceptables et éventuellement sous la forme de solvates et/ou d'hydrates.

8. Composés de formule 1 selon l'une des revendications 1 à 7, **caractérisés en ce qu'**ils se présentent sous la forme d'un de leurs sels d'addition acides avec des acides pharmacologiquement acceptables qui sont choisis dans le groupe comprenant un chlorhydrate, un bromhydrate, un iodhydrate, un hydrosulfate, un hydrophosphate, un hydrométhanesulfonate, un hydronitrate, un hydromaléate, un hydroacétate, un hydrobenzoate, un hydrocitrate, un hydrofumarate, un hydrotartrate, un hydrooxalate, un hydrosuccinate, un hydrobenzoate et un hydro-p-toluènesulfonate.

9. Composés de formule 1 selon l'une des revendications 1 à 8, **caractérisés en ce qu'**ils se présentent sous la forme des énantiomères R de formule générale *R*-1 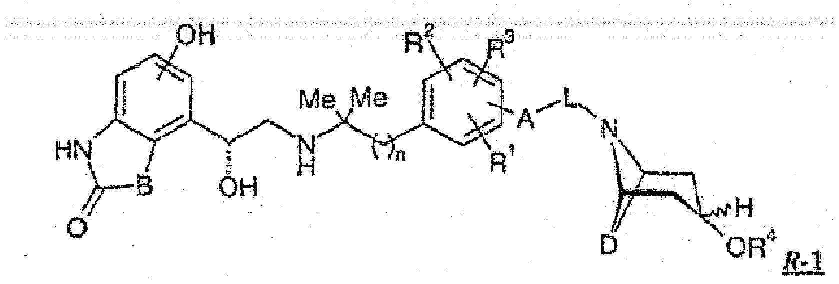 éventuellement sous la forme de leurs sels d'addition acides avec des acides pharmacologiquement acceptables et éventuellement sous la forme de solvates et/ou d'hydrates.

10. Composés de formule 1 selon l'une des revendications 1 à 8, **caractérisés en ce qu'**ils se présentent sous la forme des régio-isomères de formule générale *Régio*-1a 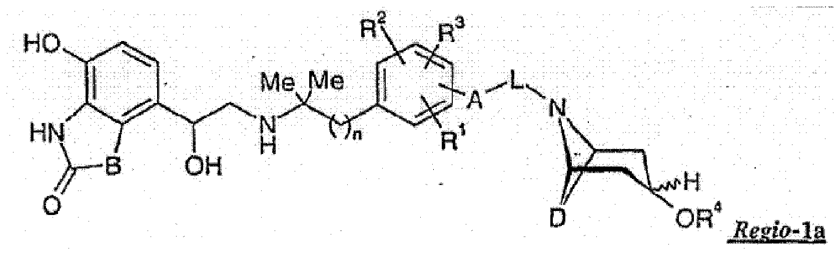 éventuellement sous la forme des énantiomères individuels ou éventuellement, des diastéréomères, de mélanges des énantiomères individuels ou éventuellement des diastéréomères ou des racémates, éventuellement sous la forme de leurs sels d'addition acides avec des acides pharmacologiquement acceptables et éventuellement sous la forme de solvates et/ou d'hydrates.

11. Composés de formule 1 selon l'une des revendications 1 à 8, **caractérisés en ce qu'**ils se présentent sous la forme des régio-isomères de formule générale *Régio*-1b 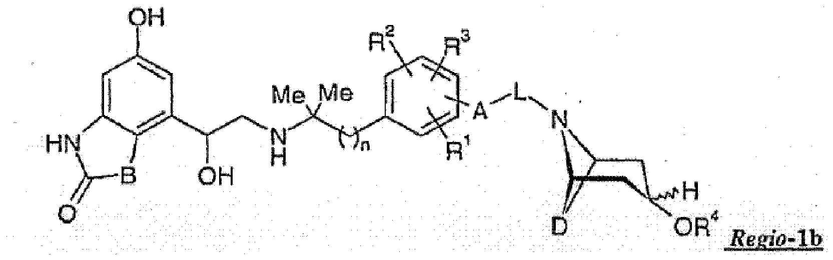 éventuellement sous la forme des énantiomères individuels ou éventuellement, des diastéréomères, de mélanges des énantiomères individuels ou éventuellement des diastéréomères ou des racémates, éventuellement sous la forme de leurs sels d'addition acides avec des acides pharmacologiquement acceptables et éventuellement sous la forme de solvates et/ou d'hydrates.

12. Composés de formule générale 1 selon l'une des revendications 1 à 11 comme médicaments.

13. Utilisation des composés de formule générale 1 selon l'une des revendications 1 à 12, pour la production d'un médicament pour le traitement des maladies des voies respiratoires.

14. Formulation pharmaceutique, **caractérisée par** une teneur en un composé de formule 1 selon l'une des revendications 1 à 12.

15. Produits intermédiaires de formule générale 4 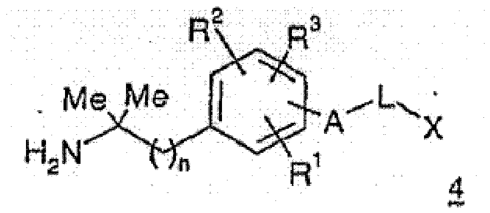 dans laquelle
X est un atome de chlore, de brome, d'iode, un groupe méthanesulfonate, trifluorométhane-sulfonate, p-toluènesulfonate ou une fonction amino protégée par un groupe protecteur approprié, de préférence, -NH-Boc, -NH-Cbz, -NH-Fmoc, -NH-Teoc ou -NH-Alloc
et dans laquelle n, A, L, R¹, R² et R³ peuvent présenter les significations mentionnées dans les revendications 1 à 8.

16. Produits intermédiaires de formule générale 5 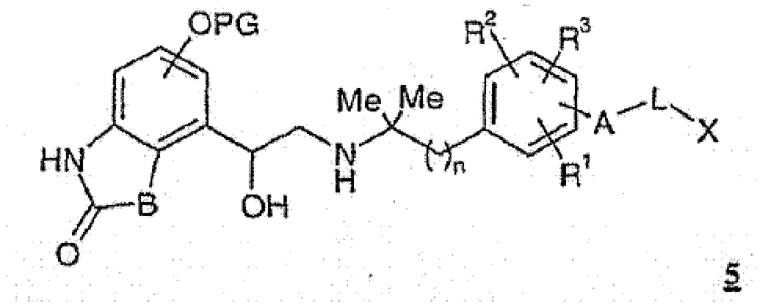 dans laquelle
OPG est une fonction hydroxy protégée par un groupe protecteur PG, de préférence, un groupe -0-alkyle en C₁ à C₄, -O-benzyle ou -O-CH₂-O-alkyle en C₁ à C₄, de préférence -0-méthyle, -O-éthyle, -O-benzyle ou -O-CH₂-O-méthyle, de manière particulièrement préférée -0-éthyle ou -0-benzyle, de manière particulièrement préférée -0-benzyle,
et dans laquelle n, A, L, X, R¹, R² et R³ peuvent présenter les significations mentionnées dans les revendications 1 à 8 et 15.

17. Produits intermédiaires de formule générale 7 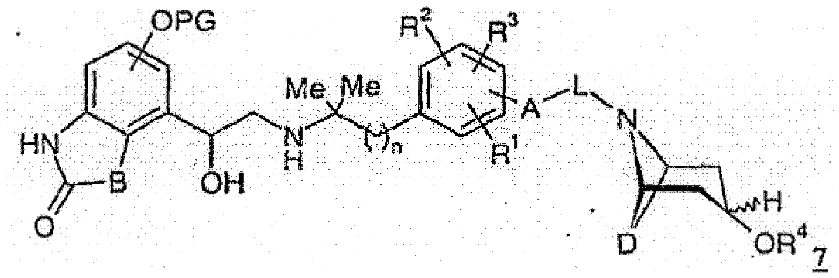 dans laquelle n, A, L, D, X, OPG, R¹, R² et R³ peuvent présenter les significations mentionnées dans les revendications 1 à 8, 15 et 16.
